# EUROPEAN PATENT SPECIFICATION

(11) **EP 3 423 046 B1**
(45) Date of publication and mention of the grant of the patent: **12.08.2020**
(21) Application number: 17706832.7
(22) Date of filing: 28.02.2017
(51) Int. Cl.: A61K 38/44, A61K 31/137, A61K 31/485, A61K 36/00, A61P 29/02, A61P 25/04, A61P 25/36, A61K 9/16, A61K 9/20

(54) **ABUSE-DETERRENT PHARMACEUTICAL COMPOSITIONS**
MISSBRAUCHSVERHINDERNDE PHARMAZEUTISCHE ZUSAMMENSETZUNGEN
COMPOSITIONS PHARMACEUTIQUES EMPÊCHANT LES ABUS

(30) Priority: 29.02.2016 EP 16157804
(43) Date of publication of application: 09.01.2019
(73) Proprietor: G.L. Pharma GmbH, 8502 Lannach (AT)
(72) Inventor: GÜBITZ, Georg, 8010 Graz (AT); GREIMEL, Katrin, 9500 Villach (AT); BRANDAUER, Martin, 9500 Villach (AT); HUBER, Daniela, 6130 Schwaz (AT); BLEYMAIER, Klaus, 1090 Viena (AT); KROUTIL, Wolfgang, 8042 Graz (AT); LECHNER, Doris, 8010 Graz (AT); WACHTER, Christof, 8502 Lannach (AT); WAGNER, Harald, 8055 Graz (AT); WINKLER, Heimo, 8045 Graz (AT)
(74) Representative: Sonn & Partner Patentanwälte
(86) International application number: PCT/EP2017/054603
(87) International publication number: WO 2017/148919

(56) References cited:
- WO-A2-2012/122422
- US-A1- 2005 176 644
- US-A1- 2011 262 359
- US-A1- 2014 330 021

## Description

The present application relates to abuse-deterrent pharmaceutical compositions comprising opioids.

Prescription opioid products are an important component of modern pain management. However, abuse and misuse of these products have created a serious and growing public health problem. One potentially important step towards the goal of creating safer opioid analgesics has been the development of opioids that are formulated to deter abuse. The development of these products is considered e.g. by the US FDA as a high public health priority (see "Abuse-Deterrent Opioids - Evaluation and Labeling"; Guidance for Industry FDA April 2015, FDA; "FDA Guidance").

Because opioid products are often manipulated for purposes of abuse by different routes of administration or to defeat extended-release (ER) properties, most abuse-deterrent technologies developed to date are intended to make manipulation more difficult or to make abuse of the manipulated product less attractive or less rewarding. It should be noted that these technologies have not yet proven successful at deterring the most common form of abuse-swallowing a number of intact capsules or tablets to achieve a feeling of euphoria. Moreover, the fact that a product has abuse-deterrent properties does not mean that there is no risk of abuse. It means, rather, that the risk of abuse is lower than it would be without such properties. Because opioid products must in the end be able to deliver the opioid to the patient, there may always be some abuse of these products.

In the FDA Guidance, abuse-deterrent properties are defined as those properties shown to meaningfully deter abuse, even if they do not fully prevent abuse. The term abuse is defined as the intentional, non-therapeutic use of a drug product or substance, even once, to achieve a desirable psychological or physiological effect. Abuse is not the same as misuse, which refers to the intentional therapeutic use of a drug product in an inappropriate way and specifically excludes the definition of abuse. The FDA Guidance uses the term "abuse-deterrent" rather than "tamper-resistant" because the latter term refers to, or is used in connection with, packaging requirements applicable to certain classes of drugs, devices, and cosmetics.

The science of abuse deterrence is relatively new, and both the formulation technologies and the analytical, clinical, and statistical methods for evaluating those technologies are rapidly evolving. The FDA Guidance identifies seven categories of abuse-deterrent formulations:
1. Physical/chemical barriers - Physical barriers can prevent chewing, crushing, cutting, grating, or grinding of the dosage form. Chemical barriers, such as gelling agents, can resist extraction of the opioid using common solvents like water, simulated biological media, alcohol, or other organic solvents. Physical and chemical barriers can limit drug release following mechanical manipulation, or change the physical form of a drug, rendering it less amenable to abuse.
2. Agonist/antagonist combinations - An opioid antagonist can be added to interfere with, reduce, or defeat the euphoria associated with abuse. The antagonist can be sequestered and released only upon manipulation of the product. For example, a drug product can be formulated such that the substance that acts as an antagonist is not clinically active when the product is swallowed (i.e. administered in the intended way and intact), but becomes active if the product is crushed and injected or snorted.
3. Aversion - Substances can be added to the product to produce an unpleasant effect if the dosage form is manipulated or is used at a higher dosage than directed. For example, the formulation can include a substance irritating to the nasal mucosa if ground and snorted.
4. Delivery System (including use of depot injectable formulations and implants) - Certain drug release designs or the method of drug delivery can offer resistance to abuse. For example, sustained-release depot injectable formulation or a subcutaneous implant may be difficult to manipulate.
5. New molecular entities and prodrugs- The properties of a new molecular entity (NME) or prodrug could include the need for enzymatic activation, different receptor binding profiles, slower penetration into the central nervous system, or other novel effects. Prodrugs with abuse-deterrent properties could provide a chemical barrier to the in vitro conversion to the parent opioid, which may deter the abuse of the parent opioid. New molecular entities and prodrugs are subject to evaluation of abuse potential for purposes of the Controlled Substances Act (CSA) .
6. Combination - Two or more of the above methods could be combined to deter abuse.
7. Novel approaches - This category encompasses novel approaches or technologies that are not captured in the previous categories.

Although there are already a number of proposals available based on the categories 1 to 5, above, there is still an unmet need to provide efficient combinations of these methods and approaches and, especially, to provide novel approaches.

The article of Rodriguez-Delgado et al. (Trends Anal. Chem. 74 (2015): p. 21-45) discloses laccase-based biosensors for the detection of phenolic compounds in food industry and for environmental and medical applications, i.a. a Clark oxygen electrode with immobilized laccase (Bauer et al., Fresenius J. Anal. Chem. 364 (1999); 179-183).

WO 98/18909 A1 discloses a transgenic organism transformed with an enzyme that is capable of transferring reducing equivalents between pyridine nucleotide cofactors.

EP 0 032 286 A2, US 3 852 157 A and US 4 391 904 A disclose methods for analysis for a member of an immunological pair using a test surface, i.a. a morphine:horseradish peroxidase conjugate and a carboxymethyl-morphine:glyoxate reductase, to detect antibodies in a sample.

WO 2012/122422 A2 and US 2011/262359 A1 disclose active agent prodrugs with heterocyclic linkers which may be cleaved enzymatically by a gastrointestinal enzyme once the prodrug has entered the gastrointestinal tract after oral ingestion. For providing the drug from the prodrug, the action of the enzyme is needed to cleave the prodrug to controllably release the drug. The provision of such prodrugs is also used to deter abuse of the actual drug.

US 2014/330021 A1 discloses aryl carboxylic acids chemically conjugated to hydromorphone as a strategy to deter abuse.

US 2005/176644 A1 and US 2012/178773 A1 disclose oxycodone derivates with a chemical moiety coupled to oxycodone to deter abuse or to substantially decrease the pharmacological activity of oxycodone.

AU 2012/201 450 A1 and WO 2006/058249 A2 refer to abuse deterrent oral dosage formulations comprising a gel forming polymer, a nasal mucosal irritating surfactant and a flushing agent together with the drug with abuse potential.

All these documents relate to common and known strategies to deter abuse of these drugs with abuse potential, such as oxycodone.

The prior art on unrelated fields discloses various compositions wherein enzymes are combined with agents or compositions for various uses:
WO 00/27204 A1 relates to an antimicrobial composition comprising an oxidoreductase and an enhancing agent of the N-hydroxyanilide-type. The compositions according to WO 00/27204 A1 are intended as detergent or cleaning compositions but also for the use as disinfectants or for the preservation of paints, food, beverages, etc..
WO 01/98518 A2 relates to methods for transforming biologically active ingredients, such as antibiotics, by laccases and manganese peroxidase enzymes. According to WO 01/98518 A2, known active ingredients should be transformed by laccases/manganese peroxidases to introduce additional functional groups to arrive at new active substances with modified properties (e.g. to overcome resistances against certain antibiotics).
EP 0 919 628 A1 discloses a method for "macromolecularizing" phenolic compounds or aromatic amine compounds by the action of enzymes with a polyphenol oxidizing activity such as laccase. EP 0 919 628 A1 therefore also discloses an enzymatic method to transform certain substances, including antimicrobial agents or viral infection inhibitors, to substances with higher molecular weight which may then be used as thickeners, stabilizers but also antimicrobial agents or viral infection inhibitors. It is also suggested to treat waste water for eliminating the phenolic compounds or aromatic amine compounds by macromolecularization. EP 0 919 628 A1 suggest laccase, catechol oxidase, polyphenol oxidase, ascorbic acid oxidase or bilirubin oxidase as enzymes which can be (industrially) used for the method disclosed.
WO 97/41215 A1 relates to industrial enzymes technology and the problem concerning storage/delivery of such enzymes. These enzymes are either delivered in dry form (where dust formation is reported to be a disadvantage) or liquid formulations (which have two disadvantages: that enzymes are not storage stable and present in their active state i.e. reacting with a substrate). In preventing this premature action with a substrate, WO 97/41215 A1 suggests to provide the enzyme and the substrate in a substantially water free liquid composition which would react in aqueous conditions but not react in the water free liquid composition.
DE 10 2006 048 833 A1 discloses a pharmaceutical preparation for the treatment of osteoporosis which comprises, in addition to collagen and a calcium-containing substance (as main active substance), a crosslinking agent which comprises crosslinking agents (for example polyhydroxyaromatics) and a catalyst of the crosslinking reaction (for example, an enzyme such as laccase) which is capable of crosslinking the crosslinking agents. The aim of DE 10 2006 048 833 A1 is to establish a three-dimensional matrix for bone support ("liquid bone").
WO 97/27841 A1 relates to stabilized enzyme formulations which can be stored and easily made available by means of a two-component dispensing system ("dispenser"). This document therefore relates generally to the provision of enzymes such as (for example) oxidoreductases or proteases, for example in cleaning fluids, shampoos or vitamin preparations.
WO 2005/063037 relates to chewing gums which may contain enzymes. The enzymes are incorporated into the chewing gum due to their catalyzing effect on degradation. Virtually all possible enzymes are listed in this document without annotation ("pairing") with other substances. The chewing gum of WO 2005/063037 may further contain a pharmaceutically, cosmetically or biologically active substance, selected from a huge list of substances without any correlation to the enzyme.
Also WO 2007/143989 A1 is drawn to a chewing gum which comprises a biodegradable polymer and (i.a.) an enzyme which is comprised in the chewing gum as a hydrophobic enzyme formulation. Again, the enzyme is provided for degrading the (biodegradable polymer in the) chewing gum. Provision of the enzyme in a hydrophobic enzyme formulation is therefore made for preventing a premature degradation.
WO 2012/003367 A2 relates to a drug delivery method, wherein a nonwoven web with a plurality of filaments contains an active agent wherein the release of the active agent from the filament may be triggered by an enzyme. This nonwoven filament is not suitable for oral ingestion.
WO 00/02464 A1 refers to a process for the treatment of tobacco, by using a phenoloxidizing enzyme in order to oxidize phenolic compounds in tobacco.

It is therefore the object of the present invention to provide a new approach and technology for abuse-deterrent drugs, especially for abuse-deterrent opioid formulations.

Therefore, the present invention provides a pharmaceutical composition comprising a drug with a laccase-reactive functional group and a laccase (EC 1.10.3.2).

Laccases (EC 1.10.3.2; CAS registry number 80498-15-3) are frequently described and analysed copper-containing oxidase enzymes that are found in many plants, fungi, and microorganisms (Riva, Trends in Biotechnology 24 (2006), 219-226). Laccases act on phenols and similar molecules, performing one-electron oxidations. According to the BRENDA database, the systematic name of laccase is benzenediol:oxygen oxidoreductase. Laccases are defined as a group of multi-copper proteins of low specificity acting on both o- and p-quinols, and often acting also on aminophenols and phenylenediamine. The semiquinone may react further either enzymatically or non-enzymatically.

Additionally, some non-laccase substrates can be oxidised by using mediators for the reaction. When these well-established laccase mediators are oxidized by laccase they generate strong oxidizing reactive species (radicals, radical quinones and quinones) which can further react with non-laccase substrates. If the laccase oxidized mediator is reduced back to its original compound, it is again re-oxidized by laccase to generate reactive species which can again oxidize another molecule. Such laccase mediators are able to oxidize molecules which cannot be oxidized directly by laccase. There are many types of mediators used in laccase formulations including aromatic centered mediators (ArOH; e.g. ferulic acid, syringaldehyde) and nitrogen centered mediators (RNOH; e.g. violuric acid, 1-hydroxybenzotriazole.

Suitable mediators are widely available in the present field. For example, mediators capable of enhancing the activity of oxidoreductases, especially of phenol-oxidizing enzymes, such as laccase, are disclosed in WO 95/01426 A1, WO 96/10079 A1 or WO 2012/085016 A1.

Another strategy involves the use of co-substrates together with laccase. Co-substrates are added to the reaction and they are oxidized by the laccase subsequently reacting and forming covalent bonds with non-laccase substrates resulting in oligomers or polymers. Generally many laccase substrates can act as cosubstrates to bind to non-laccases substrates such as phenolics (catechol), aromatic amines, alkenes, etc..

Laccases play a role in the formation of lignin by promoting the oxidative coupling of monolignols, a family of naturally occurring phenols. Laccases can be polymeric, and the enzymatically active form can be a dimer or trimer. Other laccases, such as the ones produced by the fungus Pleurotus ostreatus, play a role in the degradation of lignin, and can therefore be included in the broad category of lignin-modifying enzymes. Because laccase belongs to the oxidase enzyme family it requires oxygen as a second substrate for the enzymatic action. Spectrophotometry can be used to detect laccases, using the substrates ABTS (2,2'-azino-bis-(3-ethylbenzthiazoline-6-sulfonic acid), syringaldazine, 2,6-dimethoxyphenol, and dimethyl-p-phenylenediamine. Activity can also be monitored with an oxygen sensor, as the oxidation of the substrate is paired with the reduction of oxygen to water.

The present invention provides laccase as a novel approach for abuse-deterrent pharmaceutical drug formulations, especially opioid formulations. Such a principle has not yet been proposed or suggested in the prior art. Accordingly, no prior art composition (e.g. those referred to above) has proposed or suggested a composition comprising a drug with a laccase-reactive functional group and a laccase, wherein the drug with the laccase-reactive functional group is contained in the pharmaceutical composition together with the laccase in a storage stable, enzyme-reactive state and under conditions wherein no enzymatic activity acts on the drug with the laccase-reactive functional group.

The new strategy of the present invention is based on the use of laccase (or an enzyme system comprising laccase) which convert the drug into a non-active form (e.g. into a precipitated or inactivated product) making it non-useable (e.g. by transforming it into an inactive (or at least: less active) form or by making it impossible to inject the drug with a syringe (in vitro)). On the other hand, if the drug is administrated as foreseen (e.g. orally), proteases from the body deactivate the laccase and the drug can unfold its effect without being inactivated by the accompanying laccase. For an improved protease-degradability, the laccase may even be modified by the introduction of (additional) protease cleavage sites so as to increase the degradation rate of the laccase after exposure to such proteases.

A prerequisite of the abuse-deterrent strategy according to the present invention is that the drug has a functional group which reacts with laccase. A further prerequisite is that during storage and before use the composition according to the present invention containing a combination of the drug and laccase is kept in an environment wherein laccase does not (yet) react with the drug. This can e.g. be safeguarded by keeping (storing) both components under conditions where laccase is inactive or by spatial separation of the two components. For example, if the composition is kept in a dry form, laccase cannot react with the drug because such reaction requires aqueous conditions. As soon as a dry composition according to the present invention is dissolved in water (e.g. in order to extract the opioid drug for abuse), laccase can react with the drug and enzymatically transforms the drug into a molecule that is not (ab-)usable anymore because of its degradation or because it is polymerised. Another example for preventing the laccase to react on the drug is to spatially separate laccase from the drug so that - again - only after contact of the composition with water or a comparable solvent, laccase can react on the drug. Such spatial separation can e.g. be established by providing laccase and the drug in different compartments of the pharmaceutical formulation, by providing specific coatings, by separate granulation, etc..

On the other hand, the laccase in the compositions according to the present invention must be reactive, e.g. once exposed to aqueous environment or to other situations which are not in line with the administration routes or administration activities intended (i.e. if an abuse is likely), it has to react with the accompanied drug to prevent or at least deter abuse of this drug.

The drug contained in the composition according to the present invention has therefore to contain a functional group that can be accessed and modified by the laccase activity. Accordingly, the pharmaceutical composition according to the present invention comprises a drug, wherein the laccase-reactive functional group is a phenolic hydroxyl-group, furthermore the following organic substances can be oxidized by laccases: aminophenols, benzenethiols, di- or polyphenols, methoxy-substituted phenols, amino phenols, diamines, aromatic amines, polyamines, ascorbate, hydroxyindoles, aryl diamines, anilins, preferably substances with a phenolic hydroxyl-group (Morozova et al., Biochemistry (Moscow) 72 (2007), 1136-1150; Madhavi et al., Bioresources 4 (2009), 1694-1717; Alcalde, Industrial Enzymes, Springer Verlag, Chapter 26; Jeon et al., Trends in Biotechnology 31 (2013), 335-341). Accordingly, the drugs used according to the present invention are opioids comprising such groups and/or drugs belonging to the substance classes of benzenethiols, mono-, di- and polyphenols, methoxy-substituted phenols, aminophenols, diamines, aromatic amines, polyamines, ascorbates, hydroxyindols, aryl diamines, and anilins.

It is also possible to use reaction mediators in the composition according to the present invention which mediate the laccase reaction with the drug.

Mediators are small-molecular weight compounds that act as electron shuttle systems and broaden the substrate specificity of laccases towards more recalcitrant compounds. Often these substances cannot enter the active site of the enzyme or their redox-potential is too high. It has been shown, that aromatic contaminants like dyes, polycyclic aromatic hydrocarbons and chlorophenols were successfully detoxified as well as various organophosphorus compounds and sulfonamide antibiotics were successfully degraded using a laccase in combination with a mediator (Morozova et al., Applied Biochemistry and Microbiology, 43/5 (2007), 523-535; Canas et al., Biotechnology Advances, 28 (2010), 694-705; Johannes et al., Applied and Environmental Microbiology, 66/2 (2000), 524-528; Trovaslet-Leroy et al., Chemico-Biological Interactions, 187/1-3 (2010), 393-396; Wenig et al., Bioresource Technology, 141 (2013), 152-159) .

The laccase used in the present composition can be selected easily and optimised by availability, reactivity with the selected drug and the planned storage and administration conditions. Laccases are available from various sources, e.g. from fungi, bacteria or plants. Most of the common laccases can also be produced recombinantly. Recombinant production is preferred if consistent and continuous amounts are needed and in cases where natural sources are problematic, e.g. due to possible impurities. There are a number of laccases that are industrially used and therefore well available also in amounts necessary for mass production. For example fungal laccases are available from Collybia, Fomus, Lentinus, Pleurotus, Aspergillus, Neurospora, Podospora, Phlebia (P. radiata), Coriolus (C. hirsitus), Botrytis, Polyporus (P. pinsitus or P. versicolor, Rhizoctonia solani, Scytalidium (S. thermophilium), Pyricularia (P. oryzae), Coprinus (C. cinereus), Trametes, (T. hirsuta, T. villosa and T. versicolor), Coriolopsis (C. gallica), Phanerochaete chrysosporium, Heterobasidion annosum, Spiniger meineckellus and Myceliophthora thermophila; bacterial laccases are available from Bacillus, Pseudomonas, Streptomyces and Azospirillum. Specifically preferred laccases are from Trametes villosa, from Myceliophthora thermophila, and from Pleurotus ostreatus.

Preferred laccases are those that are already applied industrially, e.g. those used in laccase-based biosensors as reviewed in Rodriguez-Delgado et al. (Trends Anal. Chem. 74 (2015), 21-45, especially under item 2 and in tables 2 to 5 in this document). Further suitable and preferred examples of laccases are disclosed in WO 00/27204 A1, such as laccases from fungi include a laccase derivable from a strain of Aspergillus, Neurospora, e. g., N. crassa, Podospora, Botrytis, Collybia, Fomes, Lentinus, Pleurotus, Trametes, e. g., T. villosa and T. versicolor, Rhizoctonia, e. g., R. solani, Coprinus, e. g., C. cinereus, C. comatus, C. friesii, and C. plicatilis, Psathyrella, e. g., P. condelleana, Panaeolus, e. g., P. papilionaceus, Myceliophthora, e. g., M. thermophila, Schytalidium, e. g., S. thermophilum, Polyporus, e. g., P. pinsitus, Pycnoporus, e. g. P. cinnabarinus, Phlebia, e. g., P. radita (WO 92/01046 A), or Coriolus, e. g., C. hirsutus (JP 2-238885); examples from bacteria include a laccase derivable from a strain of Bacillus; laccases derived from Coprinus, Myceliophthora, Polyporus, Pycnoporus, Scytalidium or Rhizoctonia; laccases derived from Coprinus cinereus, Myceliophthora thermophila, Polyporus pinsitus, Pycnoporus cinnabarinus, Scytalidium thermophilum or Rhizoctonia solani.

A way for the determination of laccase activity (LACU) is the determination of laccase activity from the oxidation of syringaldazin under aerobic conditions. The violet colour produced is measured spectrophotometrically at 530 nm. The analytical conditions are 19 mM syringaldazin, 23 mM acetate buffer, pH 5.5, 30° C, 1 min. reaction time. 1 laccase unit (LACU) is the amount of enzyme that catalyses the conversion of 1.0 pmole syringaldazin per minute at these conditions (WO 00/27204 A1). The drug which is usable with the present invention is not critical, as long as it contains a functional group that is modifiable by the laccase activity. Of course, preferred drug to be subjected to the present new approach for abuse-deterrent products are the drugs with known abuse risk. Accordingly, preferred drugs contained in the pharmaceutical compositions according to the present invention are opioid drugs with a laccase-reactive functional group.

Opioids are substances that act on the nervous system in a similar way to opiates such as morphine and codeine. Opiates, i.e. analgesic alkaloid compounds found naturally in the opium poppy plant Papaver somniferum, therefore form a sub-group of opioids. Opioids may therefore be extracted from opium or made artificially. In the medical context, opioids are primarily used for the treatment of pain. The significant side effects of opioids, such as sedation, and a strong sense of euphoria, also expose these drugs to misuse and abuse. Opioid dependence can develop with ongoing administration, leading to a withdrawal syndrome with abrupt discontinuation. For example, morphine-like opioids are well known for their addictive properties, and for their ability to produce euphoria, motivating some to use opioids recreationally. They can cause death in overdose from respiratory depression. In 2013 between 28 and 38 million people used opioids recreationally (0.6% to 0.8% of the global population between the ages of 15 and 65; World Drug Report 2015).

Specifically preferred opioids used as drugs in the compositions according to the present invention are therefore opioids comprising a phenolic hydroxy-group, such as morphine, tapentadol, hydromorphone, etorphine, desomorphine, oxymorphone, buprenorphine, opioid peptides comprising a phenylalanine residue, such as adrenorphin, amidorphin, casomorphin, DADLE ([D-Ala², D-Leu⁵]-Enkephalin), DAMGO ([D-Ala², N-MePhe⁴, Gly-ol]-enkephalin), dermorphin, endomorphin, morphiceptin, and TRIMU 5 (L-tyrosyl-N-{[(3-methylbutyl)amino]acetyl}-D-alaninamide); oripavine, 6-MDDM (6-methylenedihydrodesoxymorphine), chlornaltrexamine, dihydromorphine, hydromorphinol, methyldesorphine, N-phenethylnormorphine, RAM-378 (7,8-Dihydro-14-hydroxy-N-phenethylnormorphine), heterocodeine, dihydroheterocodeine, 7-spiroindanyloxymorphone, morphinone, pentamorphone, semorphone, chloromorphide, nalbuphine, oxymorphazone, 1-iodomorphine, morphine-6-glucuronide, 6-monoacetylmorphine, normorphine, morphine-N-oxide, cyclorphan, dextrallorphan, levorphanol, levophenacylmorphan, norlevorphanol, oxilorphan, phenomorphan, furethylnorlevorphanol, xorphanol, butorphanol, 6,14-endoethenotetrahydrooripavine, BU-48 (N-Cyclopropylmethyl-[7α,8α,2',3']-cyclohexano-1'[S]-hydroxy-6,14-endo-ethenotetrahydronororipavine), buprenorphine, cyprenorphine, dihydroetorphine, norbuprenorphine, 5'-guanidinonaltrindole, diprenorphine, levallorphan, meptazinol, methylnaltrexone, nalfurafine, nalmefene, naloxazone, naloxone, nalorphine, naltrexone, naltriben, naltrindole, 6β-naltrexol-d4, pseudomorphine, naloxonazine, norbinaltorphimine, alazocine, bremazocine, dezocine, ketazocine, metazocine, pentazocine, phenazocine, cyclazocine, hydroxypethidine (bemidone), ketobemidone, methylketobemidone, propylketobemidone, alvimopan, picenadol and pharmaceutically acceptable salts, hydrates, solvates, esters, prodrugs and mixtures thereof, preferably morphine, tapentadol, oxymorphone, hydromorphone, buprenorphine, especially morphine. An example of an opioid drug with a phenolic amino group is anileridine.

The present pharmaceutical composition can be formulated according to the intended (non-abuse-deterrent) administration route, if necessary, adapted to the present invention (e.g. when laccase and the drug are spatially separated). Preferably, the pharmaceutical composition according to the present invention is provided as a dose unit. It is therefore preferred to provide the present composition as a tablet, e.g. a mini-tablet (i.e. small tablets with a diameter of 3mm or below), a coat-core tablet (coated tablet), a bi-layer tablet, a multi-layer tablet, a effervescent tablet, a soluble tablet, a dispersible tablet, a capsule, a pellet, a MUPS (multiple unit pellet system), a granulate, a powder, especially coated, sugar-coated and/or functionally coated (e.g. enteric coated) forms thereof. Enteric coatings are specifically advantageous for the prevention of premature inactivation of laccase, i.e. before the physiologic proteases act on the laccase. An enteric coating is generally defined as a polymer barrier applied on oral medication for protecting drugs from the acidity of the stomach. Enteric coatings are therefore usually stable at the highly acidic pH found in the stomach, but break down rapidly at a less acidic (relatively more basic; pH 7-9) pH in the intestine (i.e. after leaving the stomach). Typical substances/materials used for such enteric coatings are shellac, cellulose acetate phthalate (CAP), polyvinyl acetate phthalate (PVAP), hydroxypropylmethylcellulose phthalate, and methacrylic acid ester copolymers, or zein, for example, as well as hydrophobic or hydrophilic polymers and mixtures of such substances, if appropriate. Hydrophobic polymeric coatings include acrylic polymer, acrylic copolymer, methacrylic polymer or methacrylic copolymer, including Eudragit® L100, Eudragit® L100-55, Eudragit® L 30 D-55, Eudragit® S100, Eudragit® 4135F, Eudragit® RS, acrylic acid and methacrylic acid copolymers, methyl methacrylate, methyl methacrylate copolymers, ethoxyethyl methacrylates, cyanoethyl methacrylate, aminoalkyl methacrylate copolymer, polyacrylic acid, polymethacrylic acid, methacrylic acid alkylamine copolymer, polymethyl methacrylate, polymethacrylic acid anhydride, polymethacrylate, polyacrylamide, polymethacrylic acid anhydride and glycidyl methacrylate copolymers, an alkylcellulose such as ethylcellulose, methylcellulose, carboxymethyl cellulose, hydroxyalkylcellulose, hydroxypropyl methylcelluloses such as hydroxypropyl methylcellulose phthalate, and hydroxypropyl methylcellulose acetate succinate, cellulose acetate butyrate, cellulose acetate phthalate, and cellulose acetate trimaleate, polyvinyl acetate phthalate, polyester, waxes, shellac, zein, or the like. The coating can also include hydrophilic materials such as a pharmaceutically-acceptable, water-soluble polymer such as polyethylene oxide (PEO), ethylene oxide-propylene oxide co-polymers, polyethylene-polypropylene glycol (e.g. poloxamer), carbomer, polycarbophil, chitosan, polyvinyl pyrrolidone (PVP), polyvinyl alcohol (PVA), hydroxyalkyl celluloses such as hydroxypropyl cellulose (HPC), hydroxyethyl cellulose, hydroxymethyl cellulose and hydroxypropyl methylcellulose, carboxymethyl cellulose, sodium carboxymethyl cellulose, methylcellulose, hydroxyethyl methylcellulose, hydroxypropyl methylcellulose, polyacrylates such as carbomer, polyacrylamides, polymethacrylamides, polyphosphazines, polyoxazolidines, polyhydroxyalkylcarboxylic acids, alginic acid and its derivatives such as carrageenate alginates, ammonium alginate and sodium alginate, starch and starch derivatives, polysaccharides, carboxypolymethylene, polyethylene glycol, povidone, gelatin or the like.

Preferred enteric coating materials are methyl acrylate-methacrylic acid copolymers, cellulose acetate succinate, hydroxy propyl methyl cellulose phthalate, hydroxy propyl methyl cellulose acetate succinate, polyvinyl acetate phthalate (PVAP), methyl methacrylate-methacrylic acid copolymers, shellac, cellulose acetate trimellitate, sodium alginate and zein.

A further embodiment of the pharmaceutical composition according to the present invention is a coated composition wherein the coat comprises the laccase. The laccase acts on the drug when the coated composition is dissolved in aqueous liquids (for extracting the drugs (opioids)), whereas the laccase is immediately destroyed or inactivated after ingestion (according to the prescribed (oral) administration route) in the stomach or by the proteases in the intestine.

Since the pharmaceutical preparations according to the present invention are supposed to be administered to human patients in need of the drugs contained therein, the preparations are manufactured and finished as pharmaceutical preparations according to GMP, preferably according to the standards provided by the European Pharmacopoeia. This implies that the pharmaceutical preparations according to the present invention are marketed in properly sterile, clean and appropriately wrapped form as required by the regulatory authorities for marketed drug products, especially as required by the EMA for opioid drugs, such as morphine or oxycodone.

A specifically preferred embodiment is a composition wherein laccase is coated so as to keep the laccase inactive on the drug as long as the planned administration route is followed, i.e. which keeps the laccase activity from the drug while being coated, e.g. until excretion of the (still coated) laccase by the patient, whereas abuse activity destroys the coat so that the laccase immediately acts on the drug. For example, the laccase may be coated by one or more of the aforementioned substances/materials used for enteric coatings. Specifically preferred examples of such film-coatings or sequestering materials are Eudragit® RS (30D), Eudragit® NE 30 D; ethylcellulose, polymethacrylates (all disclosed in Rowe et al. (Eds.) "Handbook of Pharmaceutical Excipients" 6th Ed. (2009), Pharmaceutical Press, pages 262ff., 525 ff.); or the substances used in other opioid compositions for coating opioid antagonists (see e.g. EP 2 034 975 B1, US 8,465,774 B2, and WO 2004/026283 A1), except, of course and self-understanding, substances that have a degradation risk by laccase activity (e.g. because they contain a group that is converted by the laccase provided in the composition according to the present invention).

The pharmaceutical composition preferably contains the drug in the amount foreseen in the non-abuse-deterrent original composition. Accordingly, the drug is preferably contained in an amount of 0.1 to 5 000 mg (i.e. 0.1 mg to 5 g), preferably 0.5 to 1 000 mg, especially 1 to 500 mg, per dosage unit. Depending on the formulation and the expected laccase activity during normal administration, also an increase of the drug amount may be provided (compared to the non-abuse-deterrent original composition, i.e. to the pharmaceutical formulation of the drug without the abuse-deterrent features) e.g. to compensate possible loss of drug activity (if such minor loss of activity is likely or cannot be completely excluded).

The amount of laccase in the present pharmaceutical composition can be adjusted mainly based on the amount of drug, the source of laccase and the formulation details. According to a preferred embodiment, the laccase is contained in an amount of 1 to 1 000 units (i.e. 1 u to 1 ku), preferably 10 to 100 units. There are several ways to determine laccase activity. For the present invention, activities are determined by monitoring the oxidation of ABTS spectrophotometrically at 420 nm (ε= 11.4 mL µmol⁻¹ cm⁻¹) at 25°C in 100 mM sodium phosphate buffer (pH 7). The enzyme activity is expressed in Units, whereas one Unit (U) is defined as the amount of enzyme that catalyses the conversion of 1 pmole of ABTS per minute (1 U = µmol min⁻¹) (hereinafter referred to as the "laccase test according to the present invention").

The pharmaceutical compositions according to the present invention are dry compositions, usually with a moisture content (well) below 10 %, preferably with a moisture content below 5 %, especially below 2 %. The moisture content is preferably determined by accepted methods in the present field, e.g. by the European Pharmacopeia. A specifically preferred method for determination of moisture is the Karl Fischer titration method (European Pharmacopeia 8.5 (2015), 2.5.12: semi-determination of water), wherein the water of crystallisation content is included again.

Although the present invention provides a completely new strategy for abuse-deterrent drugs, the present novel approach is also combinable with other abuse-deterrent strategies, e.g. the ones that have been identified in the FDA Guidance 2015 or in Schaeffer, J. Med. Toxicol. 8 (2012), 400-407. Accordingly, the pharmaceutical composition according to the present invention preferably comprises a further abuse-deterrent feature, for example a feature selected from the group consisting of: a physical or chemical barrier, especially increased tablet hardness, a (laccase-insensitive) drug antagonist, an aversion component, an abuse-deterrent delivery system and a prodrug. Provision of a physical barrier, especially increased tablet hardness, or an aversion component, especially a gelling agent and/or a non-gelling viscosity-increasing agent (e.g. λ-carrageenan) or, e.g. an emetic or a nasal irritant, is specifically preferred. For example, the present composition can be provided as a formulation with a resistance of more than 400 N, especially more than 500 N, as prescribed in the European Pharmacopeia (Ph.Eur.8 (2014) 2.9.8). Further examples for abuse-deterrent features combinable with the present invention are the provision of discrete mechanically reinforcing particles (WO 2012/061779 A1), of materials that are both hydrophilic and hydrophobic (WO 2012/112952 A 1), of an acid soluble ingredient (a cationic polymer) with a buffering ingredient (US 9,101,636 B2), of a monolithic solidified oral dosage form prepared by a thermal process (US 2008/0075771 A1), of an emetic or a nasal irritant (US 2008/075771 A1), of an extruded formulation (US 2015/0057304 A1) or of amorphous or polymeric organic acid salts of the opioid (US 2015/016835 A1), etc..

According to a preferred embodiment the pharmaceutical composition comprises a matrix containing 1 to 80 wt.% of one or more hydrophobic or hydrophilic polymers, preferably a matrix comprising agar, alamic acid, alginic acid, carmellose, carboxymethylcellulose sodium, carbomer (such as Carbopol® carrageenan, chitosan , especially carboxymethylchitosan, catechol, copovidone, dextrin, gelatin, guar gum, hydroxyethyl cellulose, hydroxypropyl cellulose, hydroxypropyl methylcellulose, methacrylic acid copolymer, methylcellulose derivatives, microcrystalline cellulose, polyacrylic acid, polyalkylene oxide, especially polyethylene glycol, polyvinyl alcohol, polyvinyl acetate, povidone, propylene glycol alginate, a polyvinylcaprolactam-polyvinyl acetate-polyethylene glycol graft co-polymer, pullulan, silicon dioxide, sodium alginate, starch, vinylpyrrolidone-vinyl acetate copolymers, xanthan gum; or of non-polymer matrix formers like microcrystalline wax, fatty alcohols and fatty acids like stearyl alcohol, cetyl stearyl alcohol, stearic acid, palmitic acid or salts and mixtures thereof, mono-, di- and triglycerides of saturated fatty acids with a chain length between 16 and 22 carbon atoms and a mixture of such mono- di- and triglycerides, respectively.

According to a preferred embodiment of the present invention, the pharmaceutical composition comprises a hydrogel-forming component and/or suitable crosslinkers which allows the generation of insoluble crosslinked hydrogels of the drug once laccase is activated by abusive steps. Preferred hydrogel-forming components are chitosan and carboxymethylchitosan; preferred crosslinkers are phenolic crosslinkers, especially catechol and vanillin. Preferred examples of such hydrogel/crosslinker compositions are composition comprising chitosan and catechol or compositions comprising carboxymethylchitosan and vanillin.

The pharmaceutical composition according to the present invention is preferably a storage stable composition, preferably by comprising less than 5%, especially less than 1%, laccase-processed drug after 6 month storage at 25°C under dry conditions (laccase activity being determined by the laccase test according to the present invention).

In general, laccases are preferred that are acid-labile so that the laccase activity of the present composition is immediately inactivated as soon as the laccase is in contact with the stomach fluid and/or the intestine environment of the patient to whom the composition is administered.

The present pharmaceutical composition can be provided as a modified release composition, especially a prolonged release composition. The term "modified form" is meant to include accelerated release, controlled release, and sustained release forms. Certain release forms can be characterized by their dissolution profile. "Dissolution profile", means a plot of amount of active ingredient released as a function of time (Ph.Eur.8 (2014) 2.9.3). The dissolution profile can be measured utilizing the Drug Release Test <724>, which incorporates standard test USP 26 (Test <711>) of the US Pharmacopeia. A profile is characterized by the test conditions selected. Thus, the dissolution profile can be generated at a preselected apparatus, shaft speed, temperature, volume, and pH of the dissolution media. A first dissolution profile can be measured at a pH level approximating that of the stomach. A second dissolution profile can be measured at a pH level approximating that of one point in the intestine or several pH levels approximating multiple points in the intestine. A highly acidic pH may simulate the stomach and a less acidic to basic pH can simulate the intestine. By the term "highly acidic pH" it is meant a pH of about 1 to about 4. By the term "less acidic to basic pH" is meant a pH of greater than about 4 to about 7.5, preferably about 6 to about 7.5. A pH of about 1.2 can be used to simulate the pH of the stomach. A pH of about 6.0 to about 7.5, preferably about 7.5 can be used to simulate the pH of the intestine.

In contrast to modified release, "immediate release" is the conventional or non-modified release form in which greater than or equal to about 50% or more preferably about 75% of a drug according to the present invention is released e.g. within two hours of administration, preferably within one hour of administration, especially within 30 min of administration. By "controlled release" a dosage form is meant in which the drug release is controlled or modified over a period of time. Controlled can mean, for example, accelerated, sustained, delayed or pulsed release at a particular time. Alternatively, controlled can mean that the drug release is extended for longer than it would be in an immediate release dosage for, i.e., at least over several hours.

"Delayed release" indicates that there is a time-delay before significant plasma levels of the drug are achieved. A delayed release formulation according to the present invention avoids an initial burst of the drug, or can be formulated so that drug release in the stomach is avoided. A "pulsed release" formulation can contain a combination of immediate release, sustained release, and/or delayed release formulations in the same dosage form. A "semi-delayed release" formulation is a "pulsed released formulation in which a moderate dosage is provided immediately after administration and a larger dosage some hours after administration.

The terms "sustained release" or "extended release" are meant to include the release of the drug at such a rate that blood (e.g., plasma) levels are maintained within a therapeutic range but below toxic levels for at least about 8 hours, preferably at least about 12 hours, especially at least 24 hours (specifically preferred for 1 per day dosage regimen) after administration at steady-state. The term "steady-state" means that a plasma level for a given drug has been achieved and which is maintained with subsequent doses of the drug at a level which is at or above the minimum effective therapeutic level and is below the minimum toxic or overdose plasma level for a given drug. Most of the polymeric matrices and non-polymer matrix formers mentioned above have the capacity to deliver prolonged or sustained release capacity for a given drug in the formulation according to the present invention.

Specific examples for morphine retard matrices based on acrylic acid polymers are disclosed in EP 0 682 945 B1; a tapentadol retard matrix based on HPMC is disclosed in EP 1 439 829 B1.

According to a preferred embodiment, the pharmaceutical composition according to the present invention comprises the target drug alone (as the only effective ingredient) or in combination with other pharmaceutically active ingredients, preferably in combination with a non-opioid analgesic, especially with ibuprofen, diclofenac, naproxen, paracetamol and acetyl-salicylic acid.

In a preferred embodiment, the enzyme in the pharmaceutical composition essentially does not act on the drug (or any other constituents of the pharmaceutical composition) in vivo when the composition is administered in the intended way and intact (i.e. swallowed intact).

Preferably, the enzyme in the pharmaceutical composition according to the present invention is present in the composition in an essentially non-releasable form when the composition is administered in the intended way and intact (i.e. swallowed intact).

According to a preferred embodiment of the present invention the laccase enzyme in the present pharmaceutical composition is deactivated in vivo.

According to a further aspect, the present invention also relates to a method for manufacturing a pharmaceutical composition according to the present invention comprising the steps of mixing the drug with the laccase and finishing the mixture to a pharmaceutical composition. Alternatively, the method for manufacturing the pharmaceutical composition according to the present invention comprises the steps of providing the drug and the laccase in separated form and finishing the separated drug and laccase to a pharmaceutical composition.

According to another aspect, the present invention provides a composition comprising an opioid drug and a hydrogel-forming component and/or a crosslinker. The hydrogel-forming component and/or the crosslinker allow the generation of insoluble crosslinked molecules, especially as hydrogel, of the drug once the pharmaceutical composition is mixed with an aqueous mixture in the process of abusive steps. Preferred hydrogel-forming components are chitosan and carboxymethylchitosan; preferred crosslinkers are phenolic crosslinkers, especially catechol and vanillin. Preferred examples of such hydrogel/crosslinker compositions are composition comprising chitosan and catechol or compositions comprising carboxymethylchitosan and vanillin.

Preferably, the compositions according to the present inventions are provided as hydrogels. This involves the inclusion of another abuse deterrent approach, namely to create a system which changes the rheological properties of the reaction solution, i.e. the formation of a hydrogel. Hydrogels are very viscous and as a consequence, the drawing up with a needle is not possible.

The present invention also relates - by its nature - to a method of administration the (abuse-deterrent) pharmaceutical composition according to the present invention (i.e. comprising a drug with a laccase-reactive functional group and a laccase), wherein the drug with the laccase-reactive functional group is contained in the pharmaceutical composition in a storage stable, laccase-reactive state and under conditions wherein no laccase activity acts on the drug) to a patient in need thereof, wherein the pharmaceutical composition according to the present invention is orally administered to this patient in an effective amount and wherein the laccase is automatically deactivated in the course of this oral administration (by the components present in the patient's gastro-intestinal tract (mouth, esophagus, stomach, and (small) intestine(s))), e.g. by the patient's saliva, gastric juice and intestinal fluids and the patient's enzymes contained therein) so that the laccase does not act on the drug and the drug can therefore exhibit its destined effect in the patient).

The present invention is further illustrated by the following examples and the figures, yet without being restricted thereto.

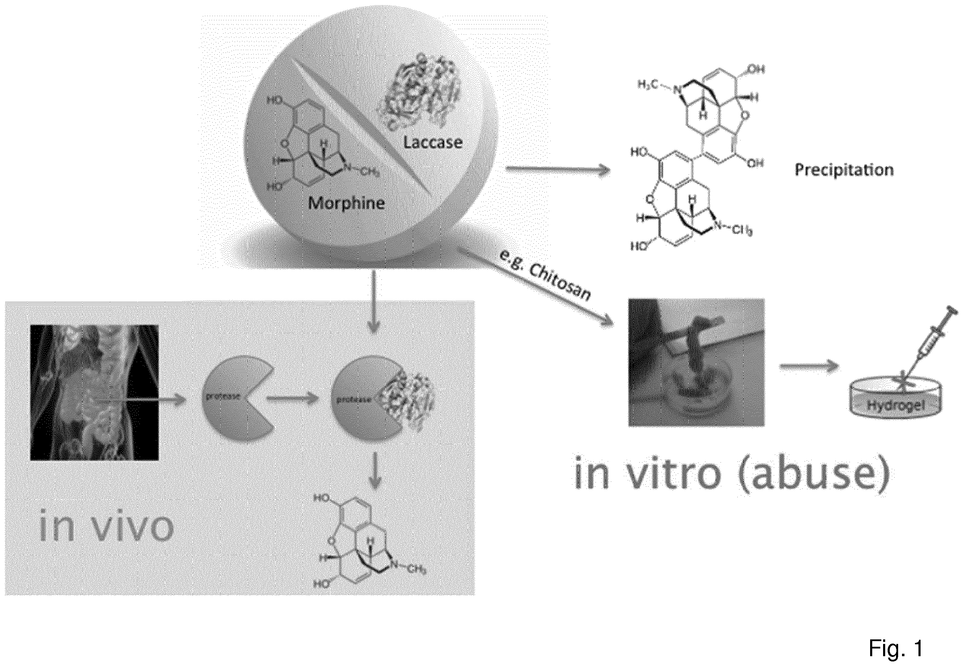

Fig. 1 shows the strategy of the present invention, exemplified by the example of morphine as drug. The abuse deterrent morphine/laccase system according to the present invention either converts morphine into a precipitated product or creates in combination with additives a hydrogel with which it is not possible anymore to inject the drug with a syringe. If the drug is administered as foreseen, proteases from the body deactivate the laccase and the drug unfolds its effect.
Fig. 2 shows the opioids that are investigated in the examples (morphine (left), tapentadol (middle) and oxycodone (right)).
Fig. 3 shows laccase oxidation of morphine with Myceliophthora thermophila (MtL) (violet) and Trametes villosa (TvL) (green).
Fig. 4 shows laccase oxidation of tapentadol with MtL (violet) and TvL (green).
Fig. 5 shows laccase oxidation of oxycodone with MtL (green) and TvL (violet).
Fig. 6 shows a TLC of morphine after enzymatic conversion at different time points compared to morphine as reference (first lane) .
Fig. 7 shows a TLC of tapentadol after enzymatic conversion at different time points compared to tapentadol as reference (first lane).
Fig. 8 shows an FTIR of morphine precipitate (red line: morphine reference, blue line: precipitated morphine polymer)
Fig. 9 shows HPLC measurement of the enzymatically oxidized morphine.
Fig. 10 shows GC-MS spectra of morphine after enzymatic conversion.
Fig. 11 shows GC-MS spectra of morphine out of a GC-MS database library; comparison of the measured spectrum to the database spectrum.
Fig. 12 shows the conversion of morphine by laccase, detected by GC-MS.
Fig. 13 shows the chitosan-catechol-morphine hydrogel.
Fig. 14 shows the carboxymethylchitosan-morphine hydrogel.
Fig. 15 shows oxygen concentration measurements with various opioids and laccase. Fig. 16 shows oxygen concentration measurement for the laccase mediator system.
Fig. 17 shows oxygen concentration measurement for the laccase mediator system and oxycodone, with the mediators vanillin, ethylvanillin, gallic acid and syringic acid.
Fig. 18 shows oxycodone concentrations in percent for various LMS experiments with oxycodone and the respective controls.
Fig. 19 shows peak area [PA] found by MS-TOF analysis for the molecule formula C₁₇H₁₉NO₄, which is oxymorphone.
Fig. 20 shows oxygen concentration measurement for the laccase mediator system and methadone, with the mediator ABTS.
Fig. 21 shows the reaction scheme for the enzymatic deacetylation of diacetylmorphine to morphine with esterases. Morphine can then in turn be oxidized by laccases.
Fig. 22 shows acetate yields over time with different enzymes and temperatures measured by HPLC/RI.
Fig. 23 shows concentrations of DAM and morphine for the assays with different enzymes and temperatures.
Fig. 24 shows inactivation of enzymes within the human body.
Fig. 25 shows SDS-Page gel showing the proteolytic degradation of different enzymes, namely Myceliophthora thermophila laccase MTL, Humicola insolens cutinase HiC and horse radish peroxidase HRP.
Fig. 26 shows pH activity profile of MTL using ABTS as substrate.
Fig. 27 shows the stability profile of MTL in different pH levels over time.
Fig. 28 shows elimination of various concentrations of morphine with 10 U/ml laccase.
Fig. 29 shows that increase of laccase concentration is linear to the decrease in morphine concentration (→ 10 times more laccase results in a ∼10 times faster degradation).
Fig. 30 shows the result with 60000 mg/L morphine, nearly at the solubility limit of morphine.

### Example:

### Laccase for use in abuse-deterrent opioid formulations

Opioids are an important component of modern pain management, though the abuse and/or misuse of those drugs has created a growing public health problem. To counteract this problem, there is a strong demand for new technologies for preventing the abuse of opioids. This project therefore tries to approach this problem with a very unique way based on using enzymes.

The potential of enzymes to polymerize opioids, thereby preventing abuse were investigated. These enzymes will not be active when opioids are administered correctly. These possibilities are thoroughly investigated by the present project.

In the present example, the development of an appropriate enzyme system to eliminate opioids from solution is shown. Moreover, optimized reaction conditions for effective conversion of the opioid solution preventing administration through injection are provided. Finally, the function of the system is verified by showing the inactivation of the opioid destroying enzymes proteolytically when the drugs are administered as foreseen.

### 1. Materials

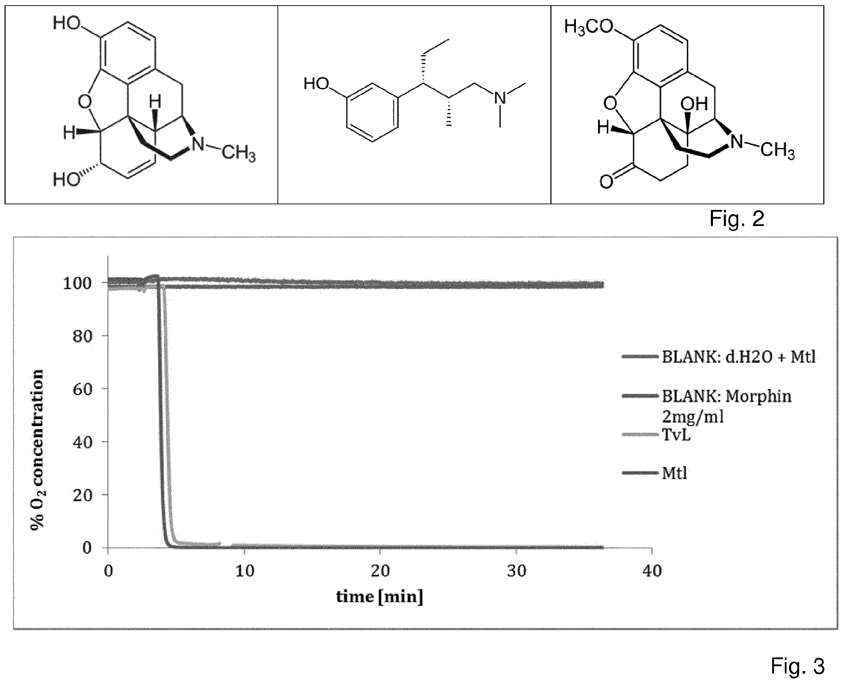

The opioids that are investigated were morphine, tapentadol and oxycodone as shown in Figure 2.

Two different laccases were used to achieve elimination of opioids - one of them originated from Trametes villosa (TvL), the other one from Myceliophthora thermophila (MtL).

### 2. Oxygen Measurements

To determine whether the laccases act on any of the given opioids (morphine, tapentadol, oxycodone), an optical oxygen sensor was used. Laccases use oxygen as electron acceptor, consequently the oxygen concentration decreases upon substrate oxidation.

The conditions for the following reactions are shown in Table 1:

**Table 1: Conditions for oxygen measurement of laccase catalysed oxidation of opioids**

| | |
|---|---|
| Enzyme (Laccase) | 10 U/ml |
| Substrate | 2 mg/ml |
| Solvent | Distilled water |
| Temperature | Room temperature |

In Figure 3 the oxidation of morphine by two different laccases namely from Myceliophthora thermophila (MtL) and Trametes villosa (TvL) is shown. The blue and red lines represent the two blanks, blue with distilled water and enzyme, and red distilled water with morphine. The violet and green lines represent the two reactions with the enzymes (violet: MtL, green: TvL). After approx. 5 minutes there is a clear decrease in the oxygen concentration in solution, meaning that the laccases are converting morphine to a reaction product. Additionally, the two solutions turned cloudy which means the reaction product precipitated. There was no significant difference between the two enzymes.

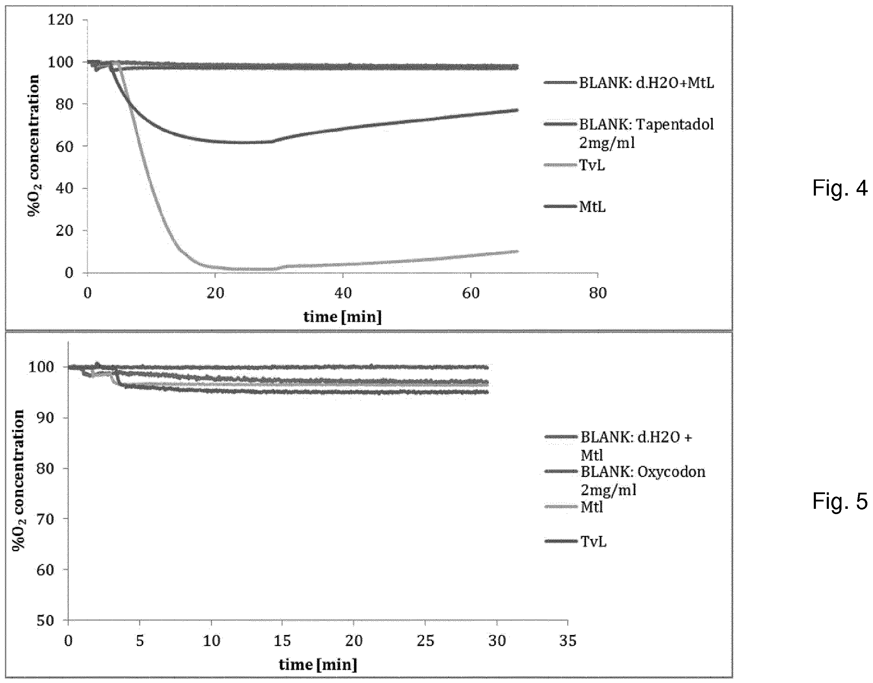

In Figure 4 the oxidation of tapentadol by MtL (violet) and TvL (green) is shown. The decrease of oxygen with MtL is much slower compared to the decrease of oxygen with TvL. This could be because TvL has a much higher redox potential than the MtL. The different curves also show that the specificity of the two enzymes is very different on tapentadol. TvL converts tapentadol better which is indicated by the slope of the curve as well as the fact that the curve in the case of TvL drops to almost 0% oxygen.

In Figure 5, the oxidation of oxycodone is shown. All of the curves look similar indicating that the two used enzymes are not capable of converting oxycodone. The cause for that is the missing phenolic - OH group in oxycodone (shown in Figure 2), which is needed by laccases.

### 3. Thin Layer Chromatography

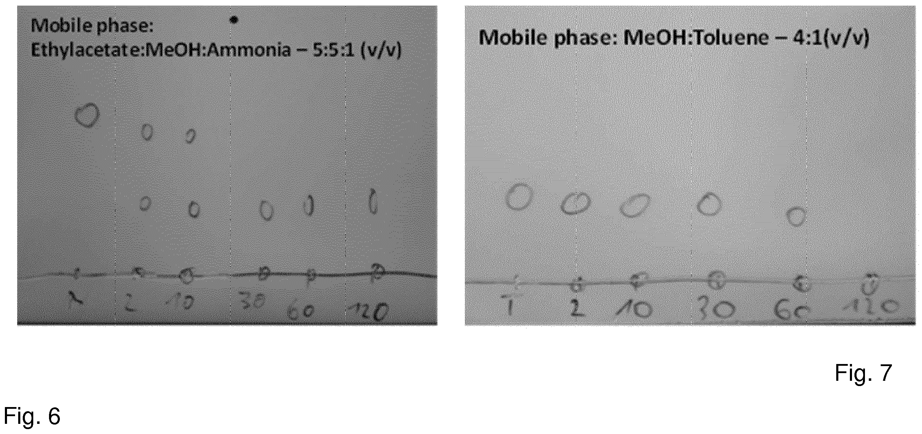

The reactions of morphine and tapentadol were also analysed by thin layer chromatography and are shown in Figure 6 and 7. Figure 6 shows the conversion of morphine. The first lane represents morphine as a reference, whereas the other lanes show samples that were taken at different time points (2, 10, 30, 60, 120 minutes). After only 2 minutes, 2 new dots are appearing on the TLC plate, indicating that a new product is formed out of the enzymatic reaction with morphine. The morphine reference dot disappears after 30 minutes, which indicates that all of the morphine is converted. The same procedure is shown in Figure 7 for tapentadol. The conversion of tapentadol is much slower, but there is also a new dot appearing after 2 minutes and the tapentadol dot is disappearing after 120 minutes.

### 4. Analysis of the precipitated product

As mentioned above the reaction product of morphine precipitated, consequently the next part was the analysis of this precipitate. The following conditions were used for the reaction (Table 2).

**Table 2: Conditions for the analysis of the morphine precipitate that is formed upon the enzymatic reaction with MtL**

| | |
|---|---|
| Enzyme | 20 U/ml |
| Substrate | 20 mg/ml |
| Solvent | Distilled water |
| Temperature | Room temperature |

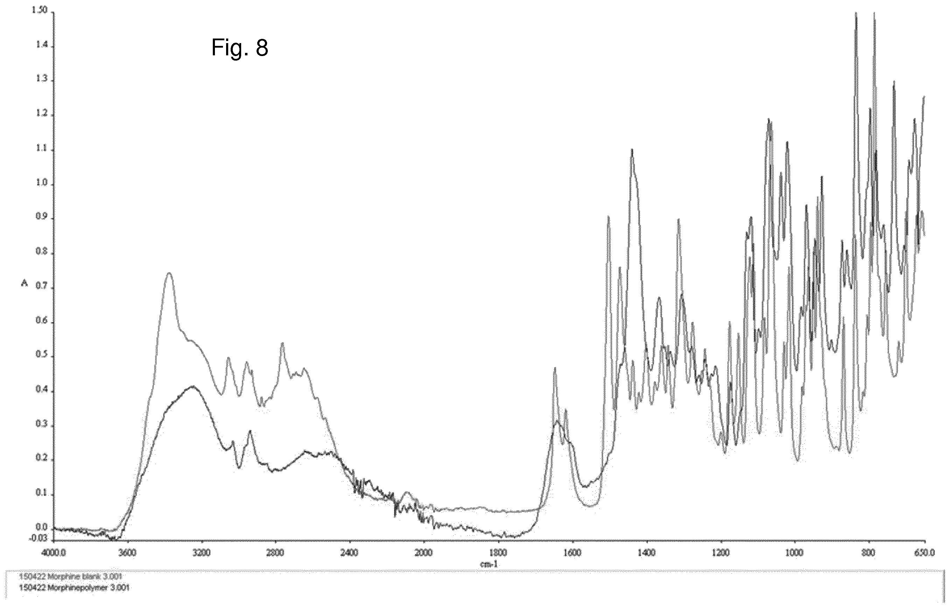

After 24 hours, the reaction mixture was centrifuged for 15 min at 16.100 rcf (relative centrifugal force). The supernatant was discarded and the remaining precipitate was lyophilized overnight. On the next day the dry precipitate was analyzed via FTIR as shown in Figure 8. The red line shows morphine, the blue line the precipitated reaction product. It can be seen that there are new peaks arising and other peaks decreasing due to the polymerization reaction of the morphine.

For further analysis a solubility test of the precipitate was performed. As indicated in Table 3, the results for the solubility test were ambivalent. In none of the used solvents the precipitate was soluble, most likely meaning that a high molecular weight product was formed upon the enzymatic reaction. This is desirable for the main goal of the project, but for further analysis a soluble compound is necessary.

**Table 3: Solubility tests of the precipitate formed upon the laccase-catalyzed oxidation of morphine**

| Solvent | Concentration of morphine precipitate [mg/ml] | Solubility |
|---|---|---|
| Acetonitrile | 0.4 | - |
| THF | 0.3 | - |
| Diethylether | 0.4 | - |
| Toluene | 0.4 | - |
| Hexane | 0.3 | - |
| 100 mM Citrate buffer pH4 | 1 | ∼ |
| 100 mM Phosphate buffer pH3 | 1 | ∼ |
| 50 mM Ammonium formate buffer pH3 | 0.001 | ∼ |

### 5. Analysis of the precipitated product

To address the second task - the changing of the rheological properties of the reaction mixture - different additives were added to increase the viscosity of the solution. The tested additives are shown in Table 4 and were mixed to the reaction as stated. Most of the chosen additives are already used in pharmaceutical applications. The desired increase in viscosity was only achieved when using hydroxypropylmethylcellulose (HPMC).

**Table 4: Additives for the enzymatic morphine reaction to increase the viscosity**

| Additive | Ratio/concentration | Troubleshooting | Increased viscosity |
|---|---|---|---|
| Ferulic acid | 1:1 | | - |
| Catechol | 1:1 | toxic | - |
| Starch | 1:1 | solubility | - |
| Polyvinylpyrrolidone (PVP) | 50 mg/ml | solubility | - |
| Polyethylenglycol (PEG6000) | 50 mg/ml | | - |
| HPMC | 100 mg/ml | time | + |
| Catechin | 1:1 | | - |
| Neohesperidin dihydrochalcon | 1:1 | | - |

### 6. Measurements of enzymatic conversion using HPLC & GC

The kinetic of the enzymatic conversion of morphine was analyzed via HPLC and GC analysis.

Samples were taken at different time points (0, 2, 5, 10, 15, 30 minutes) during the reaction. The starting solution was 0.2 mg/ml morphine in distilled water. To start the reaction, MtL was added to a final concentration of 78.3 U/ml. 100µL sample were taken and put into 900 µL of methanol (MeOH) to precipitate the enzyme. The solution was centrifuged and the supernatant was transferred to an HPLC vial via a 0.2 µm filter. Then the solution was measured with following HPLC conditions shown in Table 5.

**Table 5: Conditions for the HPLC measurement of enzymatically oxidized morphine reaction rate determination**

| | |
|---|---|
| Column | Poroshell 120 EC-C18 3.0 x 0.5mm |
| Flow | 0.8 ml/min |
| Isocratic | 85% mQ H₂O, 5% MeOH, 10% formic acid |
| Injection volume | 5 µL |
| Column temperature | 40°C |
| Signal wave length | 240 nm |

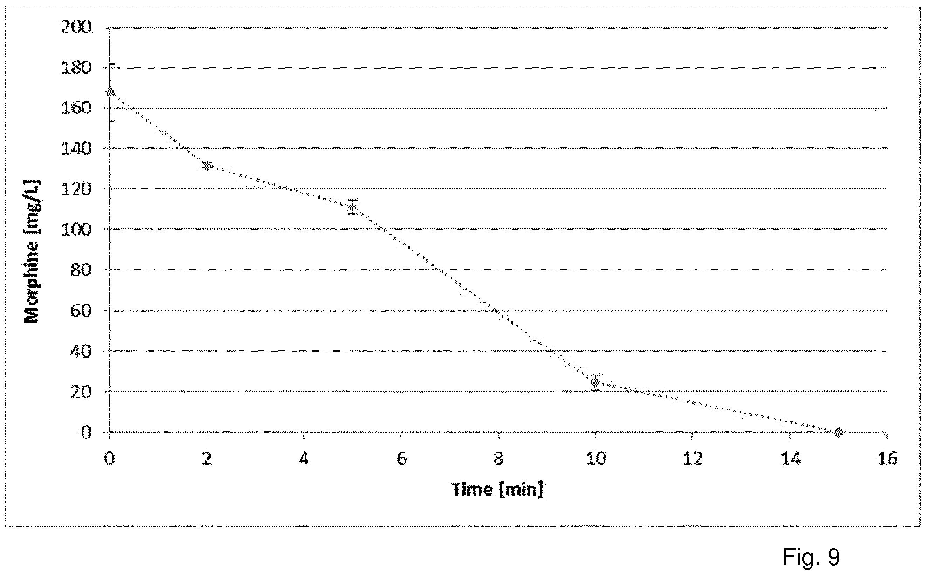

The result of the HPLC measurement is shown in Figure 9. It is clearly visible that morphine is converted by the laccase. After 15 minutes no morphine signal was measured anymore. This means after approx. 15 minutes 100% of the morphine was converted to a partly precipitated product. Further analysis of the exact reaction mechanism and reaction product has to be investigated.

In addition to the HPLC method, a GC-MS method was established and the samples were analysed using the following conditions shown in Table 6.

**Table 6: GC-MS conditions for enzymatically oxidized morphine reaction rate determination**

| | |
|---|---|
| Column | Agilent Technologies DB17MS |
| Temperature program | 120°C - 320°C |
| Run time | 11.5 min |

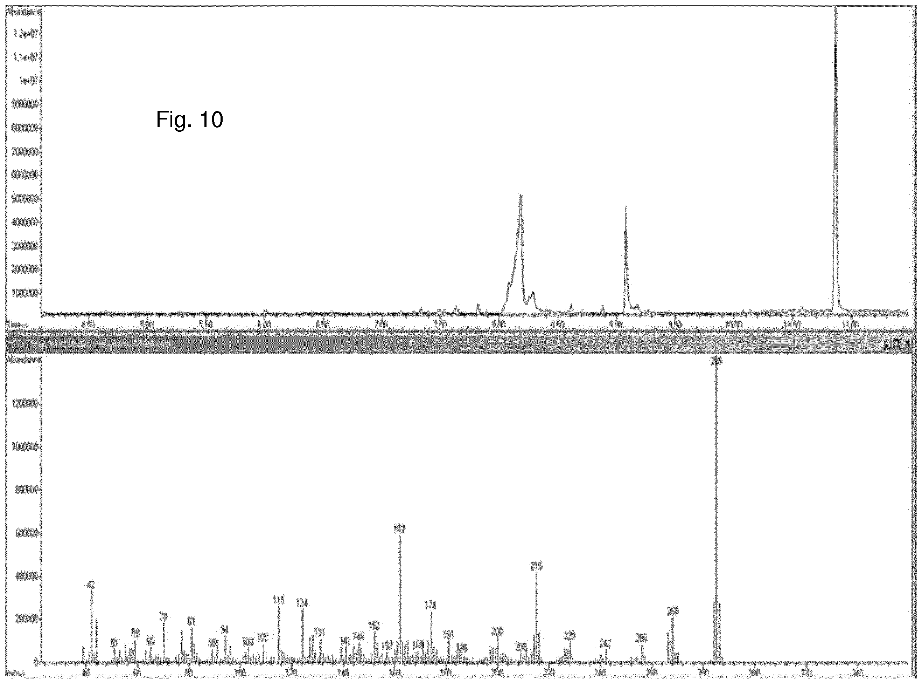

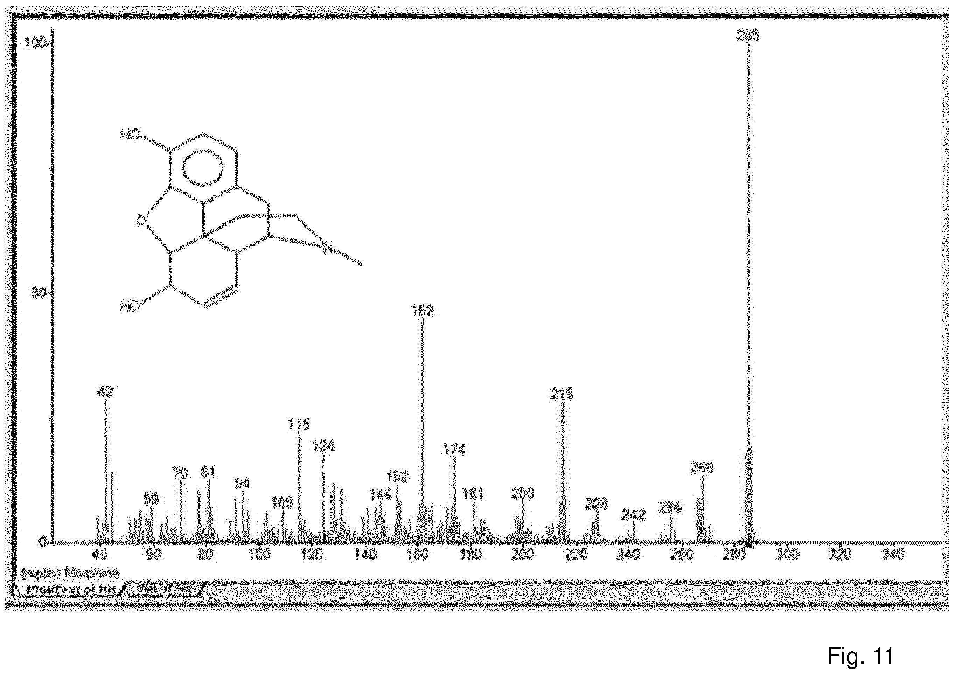

The MS spectrum in Figure 10 shows the result of the GC-MS measurement of morphine after the reaction. The sharp peak to the right represents morphine, which is confirmed by the MS spectrum library shown in figure 11. This is the proof that morphine was detected.

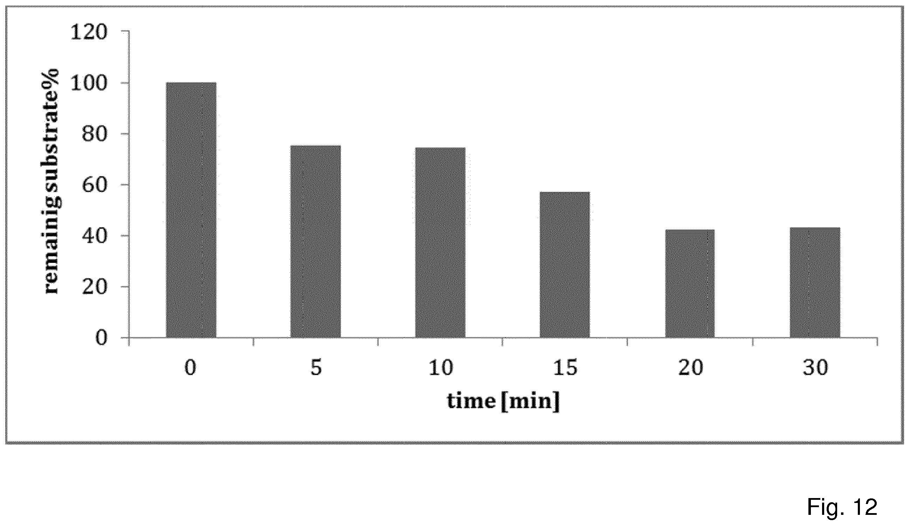

In Figure 12 the conversion rate of the reaction of morphine and laccase from *Myceliophthora thermophila* is shown. For this reaction a concentration of 2 mg/ml morphine in distilled water was used. To start the reaction MtL was added to a final concentration of 78.3 U/ml. For the sample preparation 100 µL sample were taken and added to 900 µL of methanol (MeOH) to precipitate the enzyme. The solution was centrifuged and the supernatant was transferred to an HPLC vial via a 0.2 µm filter. In the vial there was NaSO₄ to bind remaining water from the enzyme which could cause problems in the GC chromatograph.

The conversion rate, compared to the conditions of the ones mentioned above measured by HPLC, is much slower. After 30 minutes approx. 60% of the morphine is converted. The cause for this is most likely the different enzyme/substrate ratio. For the HPLC measurement, much more enzyme was used compared to the samples that were prepared for the GC measurement. This shows that it is possible to influence the conversion rate with the proportion of enzyme to substrate.

### 7. Hydrogel

According to a preferred embodiment, the compositions according to the present inventions are provided as hydrogels. This involves the inclusion of another abuse deterrent approach, namely to create a system which changes the rheological properties of the reaction solution, i.e. the formation of a hydrogel. Hydrogels are very viscous and as a consequence, the drawing up with a needle is not possible. For this purpose different set ups were tried as shown in Table 7. The substances were mixed together until a gel was formed.

The first trial with chitosan formed a hydrogel after approx. 15 minutes. The idea is that catechol crosslinks the chitosan molecules and that the morphine is covalently imbedded via Michael's type reactions. This trial was a successful proof of concept while catechol needs to be replaced with other molecules already used as drug additives.

The second trial with carboxymethylchitosan formed a hydrogel just with morphine after approx. 24h. This system would be non-harmful and the reaction time can be optimized.

| Substances | Viscosity increased |
|---|---|
| Chitosan 2% (w/v) + 500 µM Catechol + morphine 10 mg/ml + 2U/ml MtL | + (after 15 min) |
| Carboxymethylchitosan 2% (w/v) + morphine 10mg/ml + 2U MtL | + (after 24 hours) |

### Table 7: Conditions for enzymatic crosslinked hydrogels

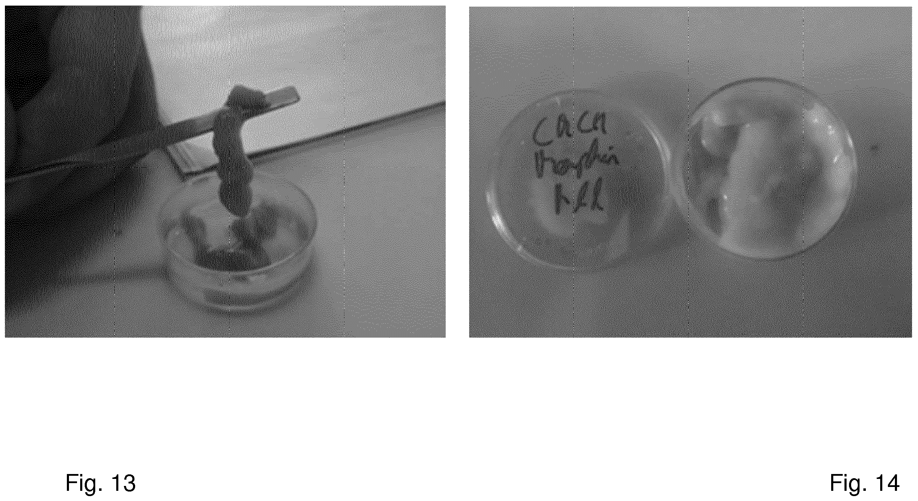

Fig. 13 shows the chitosan-catechol-morphine hydrogel. Figure 14 shows the carboxymethylchitosan-morphine hydrogel. Both hydrogels are not injectable again and cannot be used anymore for administration.

### Discussion:

This study demonstrates an entirely new enzymatic approach for the development of abuse deterrent opioids. Tapentadol and morphine were successfully converted by the enzyme laccase which was confirmed by oxygen consumption measurements, TLC, HPLC-MS, FTIR and GC-MS analysis.

Since the enzymatic conversion of morphine was quite straight forward, this reaction was chosen as a model for further analysis. The reaction rate of the laccase from Myceliophthora thermophila on morphine was analysed via HPLC and GC measurement. After approx. 15 minutes 100% of morphine was converted to a product which precipitated. The precipitated reaction product was analysed via FTIR and also solubility tests were conducted. The precipitate was hardly soluble in any of the used solvents, which is desired for abuse prevention.

Overall the desired abuse prevention system based on enzyme polymerization was successfully developed. According to the presented results it is plausible that the present system is extendable in principle to all drugs, especially all opioids that have a laccase-reactive functional group.

### 8. Additional laccase O₂ measurements

Oxygen concentration measurements were done to confirm the results that were gathered in the previous experiments. The following additional opioids were tested: methadone, dihydrocodeine, diacetylmorphine and hydromorphone.

### Final reaction mixture: 1000 mg L⁻¹ opioid, 10 Units laccase per ml in NaPi Buffer pH 7 50 mM.

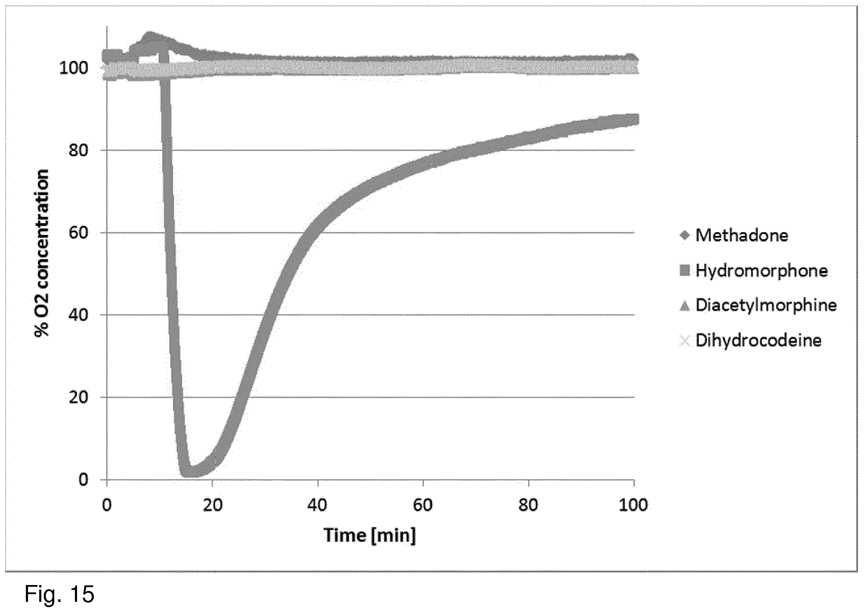

The results of the measurements can be seen in Fig. 15. The reactions confirm the previous measurements of oxycodone, tapentadol and morphine, where a drop in oxygen concentration and therefore laccase activity could only be witnessed in the presence of a phenolic group. For the newly tested opioids exclusively hydromorphone showed laccase activity, whereas with all other opioids the concentration remained steady.

### 9. Laccase mediator system (LMS): Elimination of opioids

Mediators can be used as electron shuttles to facilitate the oxidation of complex substrates that could otherwise not be oxidized by a laccase on its own. While opioids containing a phenolic group readily serve as substrate for laccases, other opioids such as oxycodone, methadone or dihydrocodeine are not as readily oxidized. The substrate range of laccases can be increased with mediators: These often small molecules are oxidized by the laccase in a first step and then react in their oxidized state with a broad variety of target substrates.

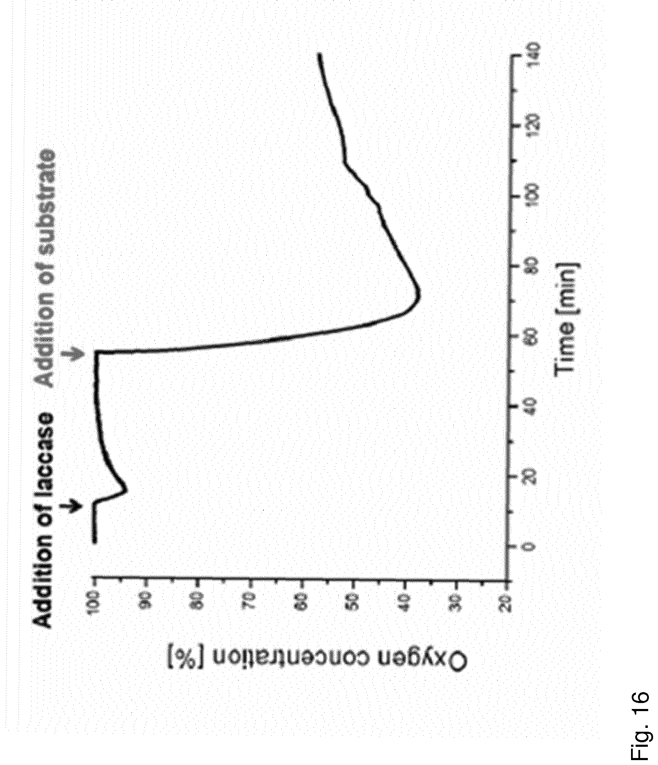

During the reaction oxygen acts as electron acceptor and is reduced to water by laccases while the mediators are oxidized. The concentration of oxygen was measured as described above. Oxygen is consumed until the mediator is fully oxidized. An open experimental setup was chosen; hence the oxygen concentration can recover to its starting value. The second step of the reaction is initiated by addition of the target substrate, which is consequently oxidized by the mediators. The now reduced mediators can again be targeted by the laccases, which leads to a decline in oxygen concentration once more (see Fig. 16).

In the present experiments the final reaction mixtures contained 0.2 to 6 mM mediator, 1-10 units of laccase per ml and 100 - 3000 mg L⁻¹ opioid.

### 9.1. LMS and Oxycodone

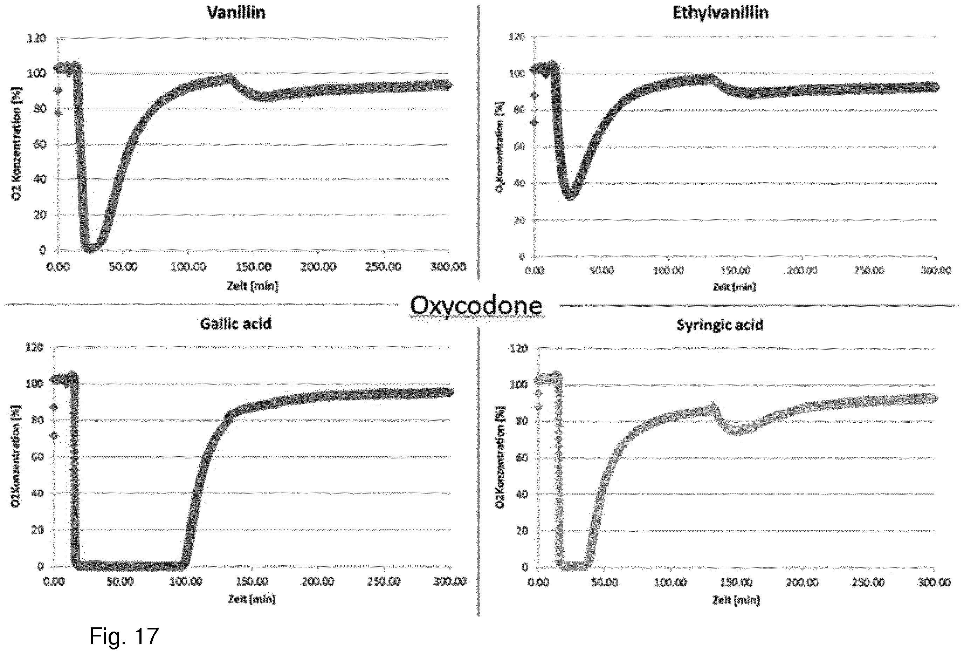

Fig. 17 shows the oxygen concentration levels for oxycodone in the laccase mediator system. The different mediators were used at concentrations of 2 mM with 10 units laccase ml⁻¹ and 1000 mgL⁻¹ oxycodone. The first drop in oxygen concentration represents the oxidation of the respective mediators with the laccase, the second drop - which took place just after the addition of the opioid - was due to the subsequent oxidation of the opioid.

The mediators vanillin, ethylvanillin and syringic acid showed a drop in oxygen concentration after the addition of oxycodone, which can be seen in Fig. 17. This shows an oxidation of oxycodone.

To further confirm the oxidation and elimination of oxycodone HPLC-MS/TOF measurements were done.

The concentrations were determined with a liquid chromatography-electrospray ionization-time of flight (HPLC-ESI-TOF) mass spectrometer from Agilent (A1260 series, Agilent US). The substances contained in the samples were separated by a Zorbax Hilic Plus, 2.1x100 mm, 3.5 µm (Agilent, US) column. The gradient was set to 100% mobile phase A (65 mM ammonium formiate pH 3.2) with a flow rate of 0.4 mL min⁻¹ at 40 °C and changed to 100% mobile phase B (acetonitrile) in 15 minutes with an injection volume of 1 µL. The spectra were acquired over the m/z range from 100 to 3000 at a scan rate of two spectra per second. The standard curve for each opioid was performed with corresponding standards ranging from 0.001 to 50 mg L⁻¹.

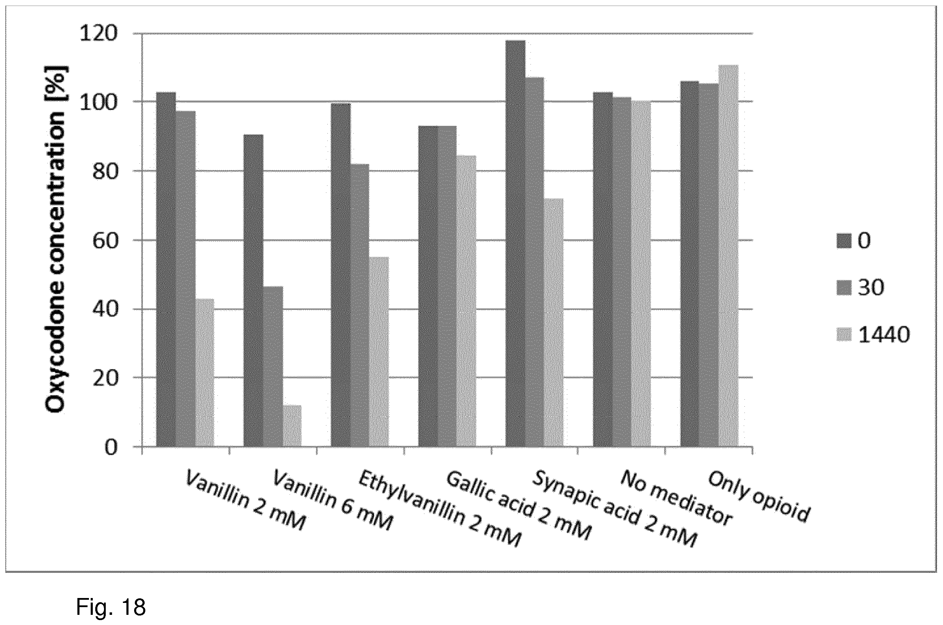

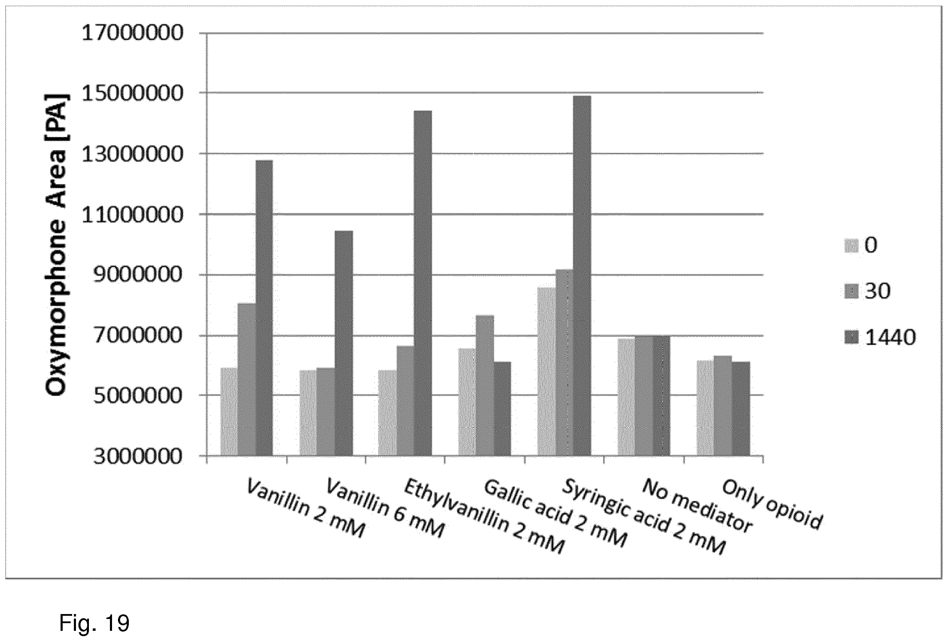

In addition to the reactions shown in Fig. 17, the mediator vanillin was used at a higher concentration (6 mM). The results can be seen in Fig. 18 and Fig. 19. The decrease in oxycodone concentration was confirmed. One of oxycodone's main degradation products, oxymorphone, was found in all samples. Oxymorphone is an impurity of oxycodone and is therefore present even in the controls where no laccase or mediator were used. However, an increase in peak area of oxymorphone over time can only be observed for oxycodone in combination with the laccase mediator system. The only mediator that had no apparent effect on oxycodone was gallic acid, an observation that was supported by both the oxygen concentration measurement and the MS-TOF analysis.

### 9.2. LMS and Methadone

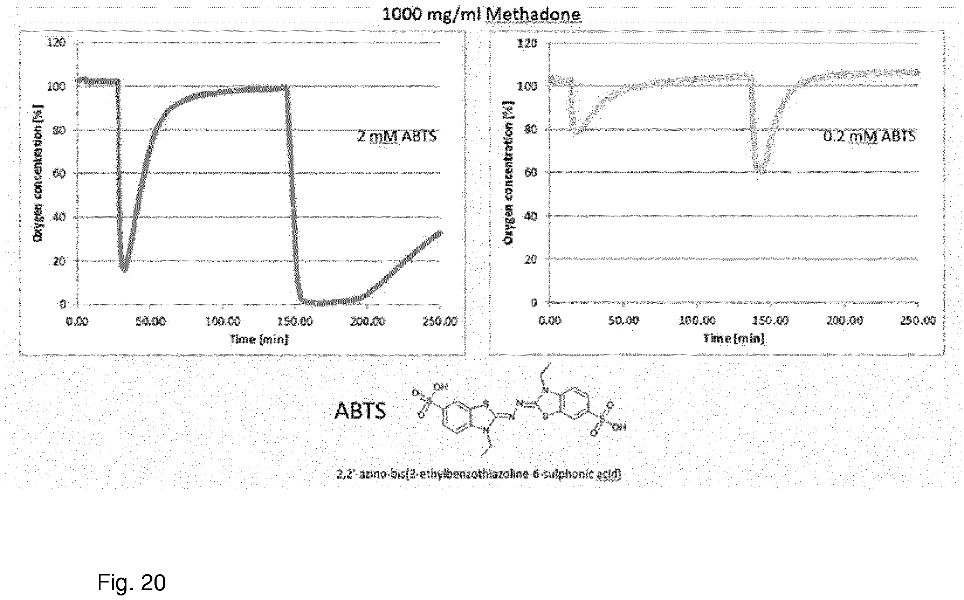

For methadone, oxygen measurements were done, but with an extended palette of mediators: In addition to the aforementioned mediators, syringic acid, TEMPO, syringol and ABTS were tested. The best effect was observed with ABTS, as can be seen in Fig. 20. Again, the decline in oxygen concentration shows oxidation of the target substance.

### 10. Diacetylmorphine (DAM) Esterase API Enzyme Couple

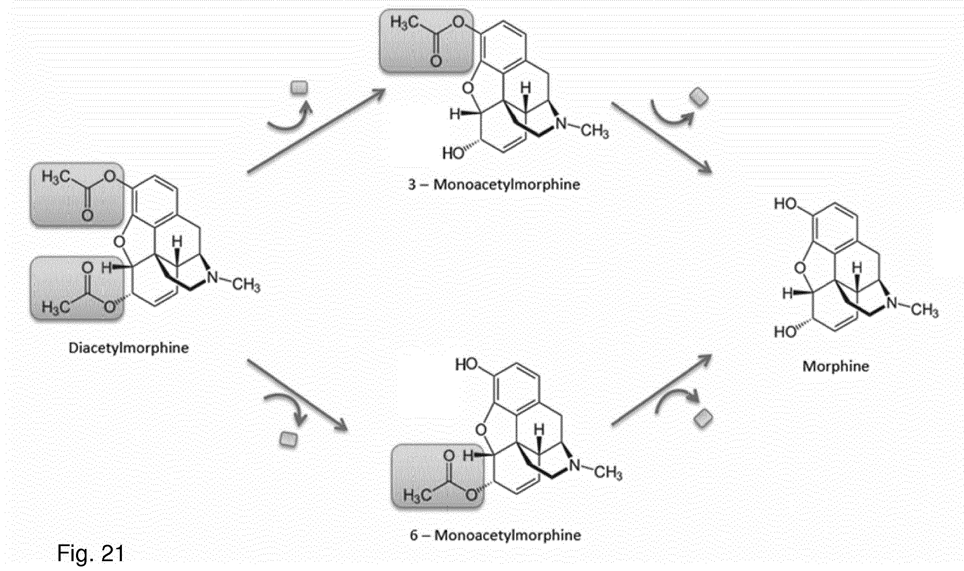

As an example for a two-step reaction for an API-enzyme couple, diacetylmorphine was investigated. In a first reaction, an esterase is used to deacetylate diacetylmorphine to monoacetylmorphine and subsequently to morphine. The general reaction scheme can be seen in Figure 21. Morphine can then be oxidized by laccases as described before.

In addition to the HPLC MS-TOF quantification the increase in acetate resulting from the deacetylation of DAM was measured by HPLC/RI.

### 10.1. Esterase activity assay

Esterase activity was determined photometrically in 50 mM sodium phosphate buffer (pH 7) using p-nitrophenol acetate (pNPA) as substrate. The method used was described by Huggins et al., J. Biol. Chem. 170 (1947) 467-482 with some modifications (Herrero Acero et al., Macromolecules 44 (2011), 4632-4640). Esterases can hydrolyze pNPA to acetic acid and p-nitrophenol. Further the increase of the absorbance of p-nitrophenol can be measured.

A stock solution of 8.3 mM pNPA was prepared in DMSO and diluted in sodium phosphate buffer. The final reaction mixture contained 200 µl of pNPA solution and 20 µl of appropriately diluted enzyme solution. The absorbance of liberated p-nitrophenol was measured at 30°C and 405 nm (ε = 90.32 mL µmol⁻¹ cm⁻¹ at pH 7) using a multimode microplate reader. One unit of esterase activity was defined as the amount of enzyme releasing 1 µmol p-nitrophenol per minute under assay conditions. For blanks the reaction mixture was prepared the same way except that the enzyme solution was substituted by water. A blank was measured using 20 µl buffer instead of sample. Volumetric enzyme activity was calculated using an adaptation of the Lambert Beer law (Hughes et al., Anal. Chem. 24 (1952), 1349-1354).

### 10.2. Enzymatic conversion of Diacetylmorphine to Morphine

*Humicola insolens* cutinase (HiC) was purchased from Novozymes (Denmark) and *Thermobifida cellulosilytica* cutinase 1 (THC) was produced and purified as described by Herrero Acero et al., 2011. These enzymes have shown to be capable of hydrolyzing complex structures in the past (Perz et al., Nat. Biotechnol. 33, (2016), 295-304). Activities of both enzymes were determined by pNPA activity assay.

The enzymatic conversion of diacetylmorphine to morphine was carried out using the two different esterases, HiC and THC. Hydrolysis reaction was performed by incubating 15 mg diacetylmorphine in 50 mM sodium phosphate buffer (pH 7) with 10 U ml⁻¹ of the respective enzyme at room temperature and at 50°C for 5 days. The final volume of the reaction mixture was 10 mL and was shaken at 400 rpm throughout the experiment. Since acetic acid is volatile the reaction had to be carried out in an airtight sealed glass tube. Samples of 1 mL at each timepoint were removed and filtered through a syringe and a membrane filter with a 0.45 µm pore size and were subsequently precipitated using the Carrez precipitation to remove the enzyme. To 1 mL of sample 20 µL of Carrez reagent 1 (10.6% w/v potassium hexacyanoferrate(II)-trihydrate) were added and vortexed. After 1 min 20 µL of C2 (28.8% w/v zinc sulfate heptahydrate) were added, vortexed and incubated for another 5 minutes followed by centrifuging for 30 minutes and 13000 rpm. The supernatant was used for further analysis.

The amount of resulting acetic acid was detected by RI-detector after separation through high performance liquid chromatography (parameters are described below). The amount of resulting acetic acid was detected by HPLC - RI analysis. For quantification acetic acid standards were prepared in a range of 1 g L⁻¹ to 0.01 g L⁻¹ in ultrapure water. Before HPLC analysis the pH of each sample had to be adjusted to 4-6 through addition of 10-20 µl of 1M HCl. All experiments were performed as duplicates. As a positive control diacetylmorphine was hydrolyzed chemically in 0.1M NaOH as described by Nakamura et al. (Nakamura et al., J. Chromatogr. 110 (1975), 81-89).

### 10.3. Quantification of deacetylation by HPLC

The obtained products were separated by high performance liquid chromatography (HPLC) from Agilent (A1100 series, Agilent US). This was done by an ICSep ION 300, 7.8x300 mm, 7 µm column supplied from Transgenomic (US). As mobile phase 0.01 N H₂SO₄ was used with a flow rate of 0.325 mL min⁻¹ at 45°C. The injection volume was 40 µL of sample at a runtime of 60 min. The amount of acetic acid was determined by the RI-detector of the instrument. The method was calibrated using acetic acid standards within a range of 10 mg L⁻¹ to 1000 mg L⁻¹.

### 10.4. Results:

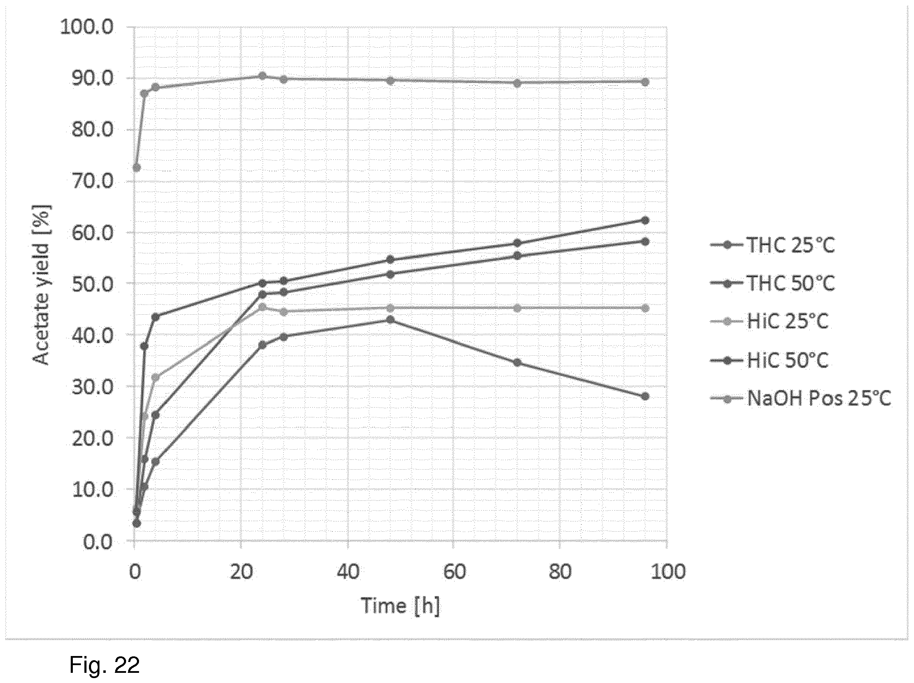

The total acetate yield over time can be seen in Fig. 22. At a temperature of 50°C both enzymes were able to hydrolyze more than 50% of the diacetylmorphine, indicating a complete deacetylation to morphine.

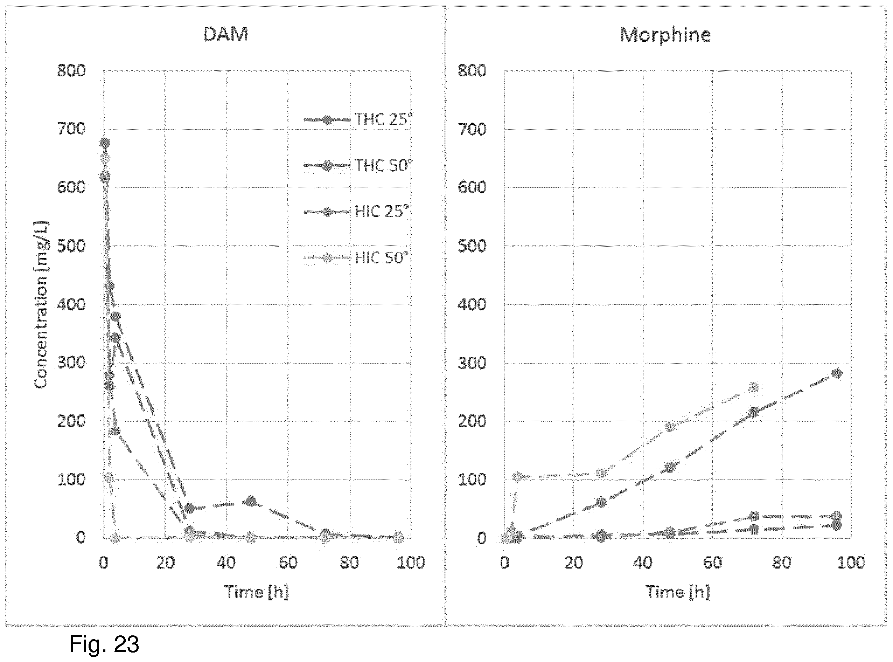

The direct measurement of opioid concentrations confirms this assumption, which can be seen in Fig. 23: While the DAM concentration declines in all samples, the morphine concentration is only increased in the samples that were incubated at 50°C. At lower temperatures, it seems that only monoacetylmorphine is produced.

### 11. Inactivation of enzymes - SGF and proteases

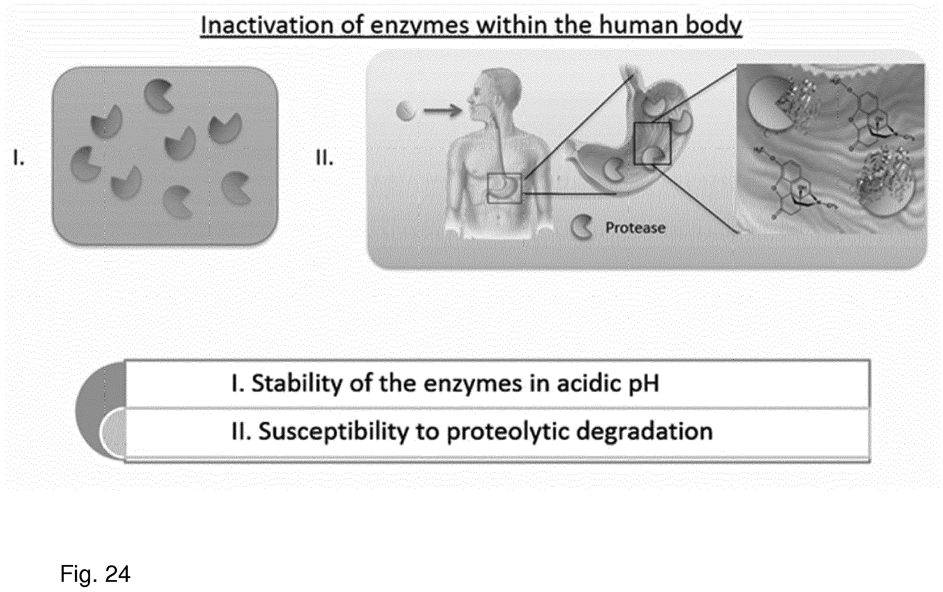

The inactivation of the enzymes when the drugs are administered as foreseen is an integral part of the invention. Inactivation can be achieved both by acidic denaturation and proteolytic degradation (Fig. 24).

To assess the feasibility of the inactivation methods, protease digestion assays as well as enzyme stability trials in various pH levels were done.

### 11.1. Pepsin digestion assay

The method used to demonstrate pepsin digestion of MTL was described by Thomas et al. (Thomas et al., Regul. Toxicol. Pharmacol. 39 (2004), 87-98) and is a standardized protocol using simulated gastric fluid (SGF). SGF contains 0.084 M HCl, 0.035 M NaCl, 4000 U of pepsin per reaction mixture and has a pH level of 1.2 according to the United States Pharmacopeia (1995). A ratio of 10000 Units of pepsin activity to 1 mg of test protein was used throughout the assay, which is based on an evaluation of the average activity of pepsin recommended in the United States Pharmacopeia (24th edition, 2000) with some modifications. Pepsin (ref. #P7000) was purchased from Sigma-Aldrich (US). All proteins that were used were dissolved in 50 mM Tris-HCl (pH 9.5) at a concentration of 5 mg mL⁻¹. The reaction mixture contained 1.52 mL of SGF preheated to 37°C before the addition of 0.08 mL of protein solution (Thomas et al., 2004). The mixture was placed into a thermomixer at 37°C and was shaken at 400 rpm. For denaturating SDS-polyacrylamide gel electrophoresis (SDS-PAGE) analysis samples of 160 µl were removed at 0 minutes, 0.5 minutes, and 15 minutes after initiation.

### 11.2 SDS-PAGE Analysis

SDS-PAGE was performed to analyze and visually inspect stained protein bands whether the protein band is still intact or fragmented after the digestion assay. Precast tris-glycin gels (ref. #456-1086) with 4-15% polyacrylamide, 10x tris-glycine-SDS (TGS) buffer (ref. #1610772) and 4x Laemmli sample buffer (ref. #1610747), were purchased from Bio-Rad Laboratories (Hercules, USA). The 4x Laemmli buffer was diluted 10:1 with 2-mercaptoethanol before usage. The following sample preparation method was described by Thomas et al (Thomas et al. 2004) .

Each 160 µl of the digestion samples was quenched by adding 56 µl of 200 mM NaHCO₃ as neutralization step and 56 µl 4 x Laemmli (Laemmli 1970) buffer for electrophoresis. Samples were immediately heated up to 99°C for 10 minutes and analyzed directly or stored at - 20 °C. Control samples for pepsin and test protein stability (SGF without pepsin but with test protein) were treated in the same way as described above. The zero time point digestion samples were prepared differently, since pepsin immediately starts to digest and auto-digest as soon as it is in solution. Before adding the test protein, pepsin was already denatured in quenching solution and heated up to 99 °C for 5 minutes. Afterwards the sample was heated again for 5 minutes to ensure proper denaturation of the test protein. 15 µl of each sample and 5 µl of prestained protein marker IV (ref. #27-2110, Peqlab) were loaded onto the gel and were subsequently run in tris-glycin running buffer for 30 - 45 minutes at 200 V. For visualization the gels were incubated with Coomassie-blue staining solution (0.1% w v⁻¹ Coomassie R250, 10%v v⁻¹ acetic acid, 45% v v⁻¹ methanol) for 30 minutes followed by a destaining step (10% v v⁻¹ acetic acid, 40% v v⁻¹ methanol) for 10-30 minutes.

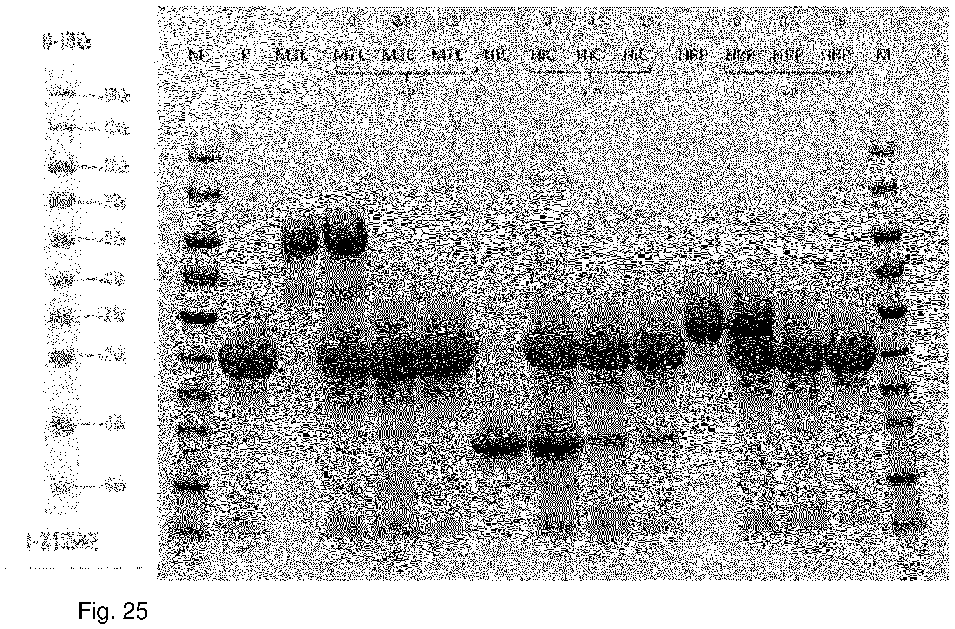

The results of the proteolytic enzyme degradation can be seen in Fig. 25. MTL and HRP are very susceptible to proteolytic degradation, as their respective protein bands vanished within 30 seconds. The HiC displayed a moderate decrease in concentration.

### 11.3 Laccase stability assay

To establish a pH profile for MTL enzyme activities were measured at different pH levels from acidic to neutral (pH 2.5 - pH 7) over time using ABTS as substrate. For every pH level MTL was diluted 1:100 in the respective buffer and was incubated for one hour and room temperature. Enzyme activity was measured at distinctive time points. Activity was measured and calculated by ABTS-assay (described above), using different extinctions coefficients for each pH level (pH7: ε = 11.38 mL µmol⁻¹ cm⁻¹, pH6: ε = 23.34 mL µmol⁻¹ cm⁻¹, pH5: ε = 32.03 mL µmol⁻¹ cm⁻¹, pH4: ε = 35.49 mL µmol⁻¹ cm⁻¹, pH3, 2: ε = 36.00 mL µmol⁻¹ cm⁻¹; master thesis ("Laccases as effective siccatives in alkyd resins"), Scholz, 2015.

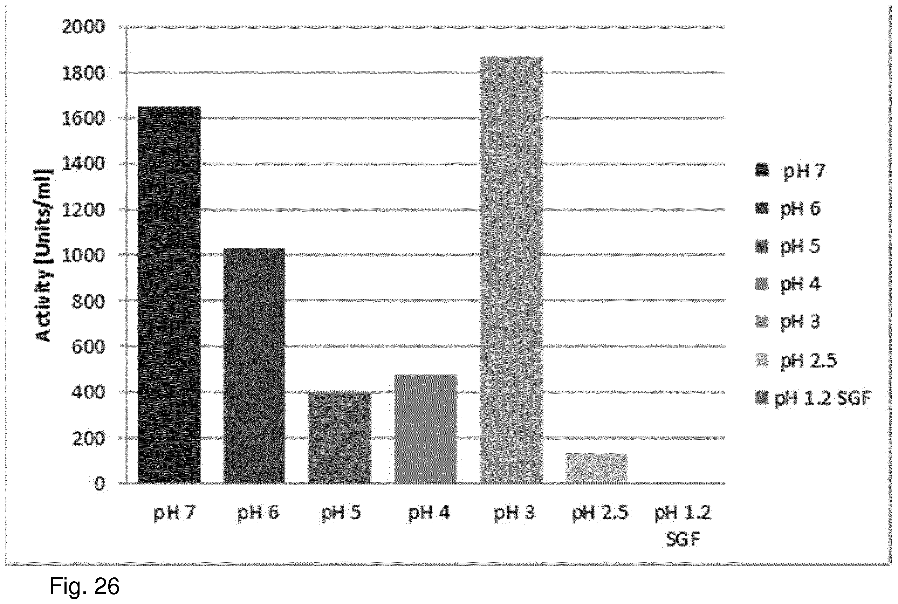

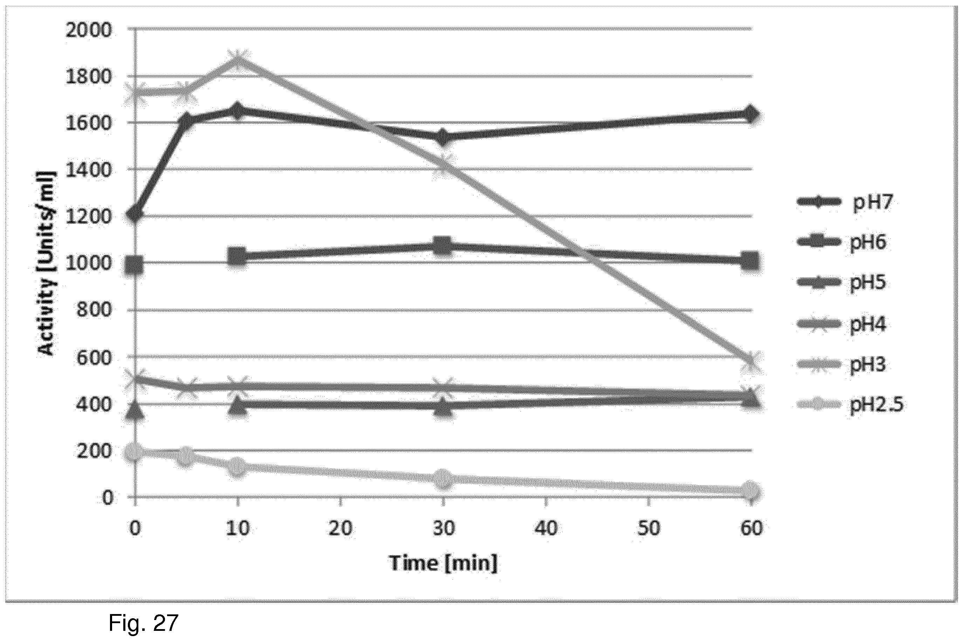

The pH profile of the MTL can be seen in Fig. 26, whereas the stability profile in different pH levels over time is shown in Fig. 27. MTL has two activity maxima, one at pH 7 and the second at pH 3 and shows no activity at SGF medium conditions. The stability profile indicates that MTL remains most of its activity at a pH range from 4 to 7 but loses activity rather quickly at lower pHs.

### 12. Additional Laccase - Morphine trials (high concentrations)

Several additional experiments were done using various concentrations of morphine and laccase. The presented results in the following examples and figures support the claims that the rate of degradation correlates directly with amount of laccase as well as the opioid concentration. The present examples are also based on an even more reliable and improved analytics.

### 12.1. Polymerization of Morphine with Laccase from Myceliophthora thermophila

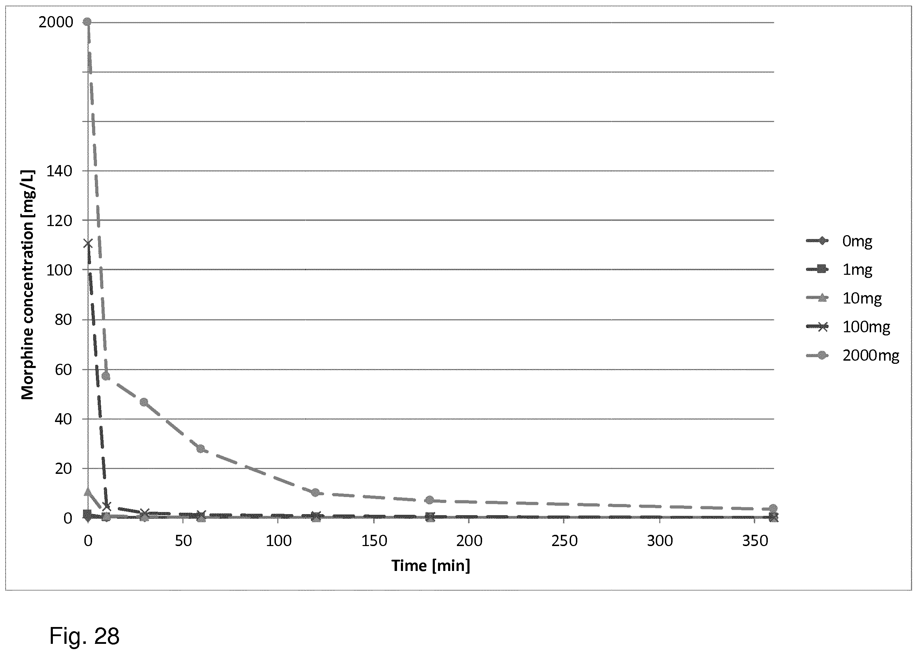

Morphine polymerization was carried out in different approaches, with morphine concentrations ranging from 1 mg l⁻¹ to 60000 mg l⁻¹. The reactions were carried out in 50 mM sodium phosphate buffer pH 7. The oxidation process was started by addition of the laccase MTL with a final activity of 10 U to 100 U. Various concentrations of morphine are shown in Fig. 28.

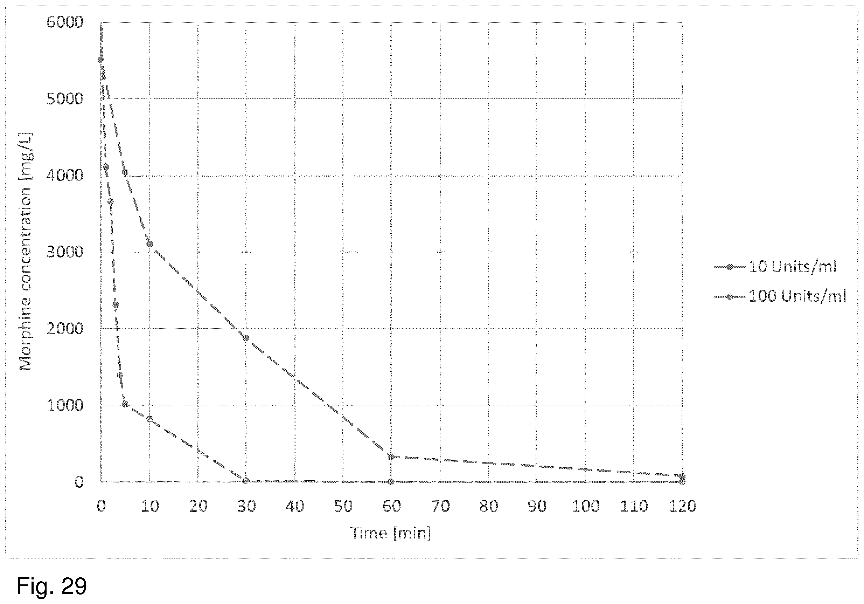

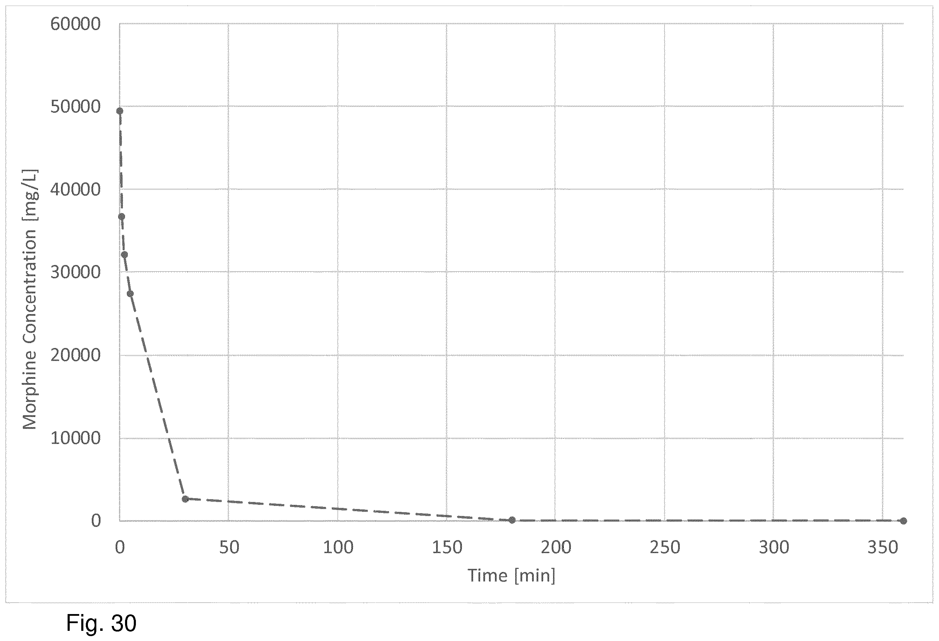

Fig. 29 shows that an increase of laccase concentration is linear to the decrease in morphine concentration (i.e. 10 times more laccase results in a ∼10 times faster degradation). Fig. 30 shows the conversion of 60000 mg/L morphine, nearly at the solubility limit of morphine.

These results are therefore fully in line with 1.6, above.

### Preferred embodiments:

The present invention therefore relates to the following preferred embodiments:
1. Pharmaceutical composition comprising a drug with a laccase-reactive functional group and a laccase (EC 1.10.3.2).
2. Pharmaceutical composition according to embodiment 1, wherein the drug with a laccase-reactive functional group is selected from the group comprising substances with a phenolic hydroxyl-group, aminophenols, benzenethiols, di- or polyphenols, methoxy-substituted phenols, amino phenols, diamines, aromatic amines, polyamines, ascorbate, hydroxyindoles, aryl diamines, and anilins, preferably substances with a phenolic hydroxyl-group.
3. Pharmaceutical composition according to embodiment 1 or 2, wherein the laccase is selected from the group of laccase from Trametes villosa, laccase from Myceliophthora thermophila, and laccase from Pleurotus ostreatus.
4. Pharmaceutical composition according to any one of embodiments 1 to 3, wherein the drug is an opioid drug with a laccase-reactive functional group, preferably selected from the group morphine, tapentadol, hydromorphone, etorphine, desomorphine, oxymorphone, buprenorphine, opioid peptides comprising a phenylalanine residue, such as adrenorphin, amidorphin, casomorphin, DADLE ([D-Ala², D-Leu⁵]-Enkephalin), DAMGO ([D-Ala², N-MePhe⁴, Gly-ol]-enkephalin), dermorphin, endomorphin, morphiceptin, and TRIMU 5 (L-tyrosyl-N-{[(3-methylbutyl)amino]acetyl}-D-alaninamide); oripavine, 6-MDDM (6-methylenedihydrodesoxymorphine), chlornaltrexamine, dihydromorphine, hydromorphinol, methyldesorphine, N-phenethylnormorphine, RAM-378 (7,8-Dihydro-14-hydroxy-N-phenethylnormorphine), heterocodeine, 7-spiroindanyloxymorphone, morphinone, pentamorphone, semorphone, chloromorphide, nalbuphine, oxymorphazone, 1-iodomorphine, morphine-6-glucuronide, 6-monoacetylmorphine, normorphine, morphine-N-oxide, cyclorphan, dextrallorphan, levorphanol, levophenacylmorphan, norlevorphanol, oxilorphan, phenomorphan, furethylnorlevorphanol, xorphanol, butorphanol, 6,14-endoethenotetrahydrooripavine, BU-48 (N-Cyclopropylmethyl-[7α,8α,2',3']-cyclohexano-1'[S]-hydroxy-6,14-endo-ethenotetrahydronororipavine), cyprenorphine, dihydroetorphine, norbuprenorphine, 5'-guanidinonaltrindole, diprenorphine, levallorphan, meptazinol, methylnaltrexone, nalfurafine, nalmefene, naloxazone, naloxone, nalorphine, naltrexone, naltriben, naltrindole, 6β-naltrexol-d4, pseudomorphine, naloxonazine, norbinaltorphimine, alazocine, bremazocine, dezocine, ketazocine, metazocine, pentazocine, phenazocine, cyclazocine, hydroxypethidine (bemidone), ketobemidone, methylketobemidone, propylketobemidone, alvimopan, picenadol and pharmaceutically acceptable salts, hydrates, solvates, esters, prodrugs and mixtures thereof, more preferred morphine, tapentadol, buprenorphine, oxymorphone, pentazocine, levorphanol, hydromorphone, especially morphine.
5. Pharmaceutical composition according to any one of embodiments 1 to 4, wherein the pharmaceutical composition is selected from a tablet, a mini-tablet, a coat-core tablet (coated tablet), a bi-layer tablet, a multi-layer tablet, a capsule, a pellet, a MUPS (multiple unit pellet system), a granulate, a powder, especially coated, sugar-coated and/or functionally coated (e.g. enteric coated) forms thereof.
6. Pharmaceutical composition according to any one of embodiments 1 to 5, wherein the composition comprises a coated laccase.
7. Pharmaceutical composition according to any one of embodiments 1 to 6, wherein the drug is contained in an amount of 0.1 to 5 000 mg, preferably 0.5 to 1 000 mg, especially 1 to 500 mg, per dosage unit.
8. Pharmaceutical composition according to any one of embodiments 1 to 7, wherein the laccase is contained in an amount of 1 to 1 000 units, preferably 10 to 100 units.
9. Pharmaceutical composition according to any one of embodiments 1 to 8, wherein the composition comprises a further abuse-deterrent feature, preferably selected from the group a physical or chemical barrier, especially increased tablet hardness, a drug antagonist, an aversion component, an abuse-deterrent delivery system and a prodrug, especially a physical barrier or an aversion component, especially a gelling agent and/or a non-gelling viscosity-increasing agent.
10. Pharmaceutical composition according to any one of embodiments 1 to 9, wherein the composition comprises a matrix containing 1 to 80 wt.% of one or more hydrophobic or hydrophilic polymers, preferably a matrix comprising agar, alamic acid, alginic acid, carmellose, carboxymethylcellulose sodium, carbomer, carrageenan, chitosan, especially carboxymethylchitosan, catechol , copovidone, dextrin, gelatin, hydroxyethyl cellulose, hydroxypropyl cellulose, hydroxypropyl methylcellulose, methacrylic acid copolymers, methylcellulose derivatives, microcrystalline cellulose, polyacrylic acid, polyalkylene oxide, especially polyethylene glycol, polyvinyl alcohol, polyvinyl acetate, povidone, propylene glycol alginate, a polyvinylcaprolactam-polyvinyl acetate-polyethylene glycol graft co-polymer, pullulan, silicon dioxide, sodium alginate, starch, vinylpyrrolidone-vinyl acetate copolymers or of a non-polymer matrix former, preferably microcrystalline wax, fatty alcohols and fatty acids, especially stearyl alcohol, cetyl stearyl alcohol, stearic acid, palmitic acid or salts and mixtures thereof, mono-, di- and triglycerides of saturated fatty acids with a chain length between 16 and 22 carbon atoms and a mixture of such mono- di- and triglycerides.
11. Pharmaceutical composition according to any one of embodiments 1 to 10, wherein the composition is storage stable, preferably by comprising less than 5%, especially less than 1%, laccase-processed drug after 6 month storage at 25°C under dry conditions.
12. Pharmaceutical composition according to any one of embodiments 1 to 11, further comprising a hydrogel-forming component and/or a crosslinker, preferably chitosan and/or catechol or carboxymethylchitosan and/or vanillin.
13. Pharmaceutical composition according to any one of embodiments 1 to 12, wherein the composition is a modified release composition, especially a prolonged release composition.
14. Pharmaceutical composition according to any one of embodiments 1 to 13, wherein the composition renders immediate release, modified release, or a combination thereof.
15. Pharmaceutical composition according to any one of embodiments 1 to 14, wherein the composition comprises an opioid analgesic alone or in combination with a non-opioid analgesic, especially with ibuprofen, diclofenac, naproxen, paracetamol and acetyl-salicylic acid.
16. Pharmaceutical composition according to any one of embodiments 1 to 15 for use in the treatment of drug addiction.
17. Pharmaceutical composition according to any one of embodiments 1 to 15 for use in the treatment of pain.
18. Pharmaceutical composition according to any one of embodiments 1 to 15, wherein the drug-processing enzyme essentially does not act on the drug in vivo when the composition is administered in the intended way and intact.
19. Pharmaceutical composition according to any one of embodiments 1 to 15, wherein the drug-processing enzyme exists in the formulation in an essentially non-releasable form when the composition is administered in the intended way and intact.
20. Pharmaceutical composition according to any one of embodiments 1 to 15, wherein the drug-processing enzyme is deactivated in vivo.
21. Pharmaceutical compositions according to any one of embodiments 1 to 20, with a moisture content below 10 %, preferably with a moisture content of below 5 %, especially with a moisture content of below 2 %.
22. Method for manufacturing a pharmaceutical composition according to any one of embodiments 1 to 21 comprising the steps of mixing the drug with the laccase and finishing the mixture to a pharmaceutical composition.
23. Method for manufacturing a pharmaceutical composition according to any one of embodiments 1 to 21 comprising the steps of providing the drug and the laccase in separated form and finishing the separated drug and laccase to a pharmaceutical composition.
24. Method of administration the pharmaceutical composition according to any one of embodiments 1 to 21 to a patient in need thereof, wherein the pharmaceutical composition is orally administered to this patient in an effective amount and wherein the laccase is automatically deactivated in the course of this oral administration.

## Claims

1. Pharmaceutical composition comprising a drug with a laccase-reactive functional group and a laccase (EC 1.10.3.2).

2. Pharmaceutical composition according to claim 1, wherein the drug with a laccase-reactive functional group is selected from the group comprising substances with a phenolic hydroxyl-group, aminophenols, benzenethiols, di- or polyphenols, methoxy-substituted phenols, amino phenols, diamines, aromatic amines, polyamines, ascorbate, hydroxyindoles, aryl diamines, and anilins,, preferably substances with a phenolic hydroxyl-group.

3. Pharmaceutical composition according to claim 1 or 2, wherein the laccase is selected from the group of laccase from Trametes villosa, laccase from Myceliophthora thermophila, and laccase from Pleurotus ostreatus.

4. Pharmaceutical composition according to any one of claims 1 to 3, wherein the drug is an opioid drug with a laccase-reactive functional group, preferably selected from the group morphine, tapentadol, hydromorphone, etorphine, desomorphine, oxymorphone, buprenorphine, opioid peptides comprising a phenylalanine residue, such as adrenorphin, amidorphin, casomorphin, DADLE ([D-Ala², D-Leu⁵]-Enkephalin), DAMGO ([D-Ala², N-MePhe⁴, Gly-ol]-enkephalin), dermorphin, endomorphin, morphiceptin, and TRIMU 5 (L-tyrosyl-N-{[(3-methylbutyl)amino]acetyl}-D-alaninamide); oripavine, 6-MDDM (6-methylenedihydrodesoxymorphine), chlornaltrexamine, dihydromorphine, hydromorphinol, methyldesorphine, N-phenethylnormorphine, RAM-378 (7,8-Dihydro-14-hydroxy-N-phenethylnormorphine), heterocodeine, 7-spiroindanyloxymorphone, morphinone, pentamorphone, semorphone, chloromorphide, nalbuphine, oxymorphazone, 1-iodomorphine, morphine-6-glucuronide, 6-monoacetylmorphine, normorphine, morphine-N-oxide, cyclorphan, dextrallorphan, levorphanol, levophenacylmorphan, norlevorphanol, oxilorphan, phenomorphan, furethylnorlevorphanol, xorphanol, butorphanol, 6,14-endoethenotetrahydrooripavine, BU-48 (N-Cyclopropylmethyl-[7α,8α,2',3']-cyclohexano-1'[S]-hydroxy-6,14-endo-ethenotetrahydronororipavine), cyprenorphine, dihydroetorphine, norbuprenorphine, 5'-guanidinonaltrindole, diprenorphine, levallorphan, meptazinol, methylnaltrexone, nalfurafine, nalmefene, naloxazone, naloxone, nalorphine, naltrexone, naltriben, naltrindole, 6β-naltrexol-d4, pseudomorphine, naloxonazine, norbinaltorphimine, alazocine, bremazocine, dezocine, ketazocine, metazocine, pentazocine, phenazocine, cyclazocine, hydroxypethidine (bemidone), ketobemidone, methylketobemidone, propylketobemidone, alvimopan, picenadol and pharmaceutically acceptable salts, hydrates, solvates, esters, prodrugs and mixtures thereof, more preferred morphine, tapentadol, buprenorphine, oxymorphone, pentazocine, levorphanol, hydromorphone, especially morphine.

5. Pharmaceutical composition according to any one of claims 1 to 4, wherein the pharmaceutical composition is selected from a tablet, a mini-tablet, a coat-core tablet (coated tablet), a bi-layer tablet, a multi-layer tablet, a capsule, a pellet, a MUPS (multiple unit pellet system), a granulate, a powder, especially coated, sugar-coated and/or functionally coated (e.g. enteric coated) forms thereof.

6. Pharmaceutical composition according to any one of claims 1 to 5, wherein the composition comprises a coated laccase.

7. Pharmaceutical composition according to any one of claims 1 to 6, wherein the drug is contained in an amount of 0.1 to 5 000 mg, preferably 0.5 to 1 000 mg, especially 1 to 500 mg, per dosage unit.

8. Pharmaceutical composition according to any one of claims 1 to 7, wherein the laccase is contained in an amount of 1 to 1.000 units, preferably 10 to 100 units.

9. Pharmaceutical composition according to any one of claims 1 to 8, wherein the composition comprises a further abuse-deterrent feature, preferably selected from the group a physical or chemical barrier, especially increased tablet hardness, a drug antagonist, an aversion component, an abuse-deterrent delivery system and a prodrug, especially a physical barrier or an aversion component, especially a gelling agent and/or a non-gelling viscosity-increasing agent.

10. Pharmaceutical composition according to any one of claims 1 to 9, wherein the composition comprises a matrix containing 1 to 80 wt.% of one or more hydrophobic or hydrophilic polymers, preferably a matrix comprising agar, alamic acid, alginic acid, carmellose, carboxymethylcellulose sodium, carbomer, carrageenan, chitosan, especially carboxymethylchitosan, catechol, copovidone, dextrin, gelatin, hydroxyethyl cellulose, hydroxypropyl cellulose, hydroxypropyl methylcellulose, methacrylic acid copolymers, methylcellulose derivatives, microcrystalline cellulose, polyacrylic acid, polyalkylene oxide, especially polyethylene glycol, polyvinyl alcohol, polyvinyl acetate, povidone, propylene glycol alginate, a polyvinylcaprolactam-polyvinyl acetate-polyethylene glycol graft co-polymer, pullulan, silicon dioxide, sodium alginate, starch, vinylpyrrolidone-vinyl acetate copolymers or of a non-polymer matrix former, preferably microcrystalline wax, fatty alcohols and fatty acids, especially stearyl alcohol, cetyl stearyl alcohol, stearic acid, palmitic acid or salts and mixtures thereof, mono-, di- and triglycerides of saturated fatty acids with a chain length between 16 and 22 carbon atoms and a mixture of such mono- di- and triglycerides.

11. Pharmaceutical composition according to any one of claims 1 to 10, wherein the composition is storage stable, preferably by comprising less than 5%, especially less than 1%, laccase-processed drug after 6 month storage at 25°C under dry conditions.

12. Pharmaceutical composition according to any one of claims 1 to 11, further comprising a hydrogel-forming component and/or a crosslinker, preferably chitosan and/or catechol or carboxymethylchitosan and/or vanillin.

13. Method for manufacturing a pharmaceutical composition according to any one of claims 1 to 12 comprising the steps of mixing the drug with the laccase and finishing the mixture to a pharmaceutical composition.

14. Method for manufacturing a pharmaceutical composition according to any one of claims 1 to 12 comprising the steps of providing the drug and the laccase in separated form and finishing the separated drug and laccase to a pharmaceutical composition.

15. Pharmaceutical composition according to any one of claims 1 to 12 for use in the treatment of pain and/or drug addiction.

## Patentansprüche

1. Pharmazeutische Zusammensetzung, aufweisend einen Wirkstoff mit einer Laccase-reaktiven funktionellen Gruppe und eine Laccase (EC 1.10.3.2).

2. Pharmazeutische Zusammensetzung nach Anspruch 1, wobei der Wirkstoff mit einer Laccase-reaktiven funktionellen Gruppe aus der Gruppe ausgewählt ist, aufweisend Substanzen mit einer phenolischen Hydroxylgruppe, Aminophenole, Benzenthiole, Di- oder Polyphenole, methoxysubstituierte Phenole, Aminophenole, Diamine, aromatische Amine, Polyamine, Ascorbat, Hydroxyindole, Aryldiamine und Aniline, vorzugsweise Substanzen mit einer phenolischen Hydroxylgruppe.

3. Pharmazeutische Zusammensetzung nach Anspruch 1 oder 2, wobei die Laccase aus der Gruppe von Laccase aus Trametes villosa, Laccase aus Myceliophthora thermophila und Laccase von Pleurotus ostreatus ausgewählt ist.

4. Pharmazeutische Zusammensetzung nach einem der Ansprüche 1 bis 3, wobei der Wirkstoff ein opioider Wirkstoff mit einer Laccase-reaktiven funktionellen Gruppe ist, vorzugsweise ausgewählt aus der Gruppe, bestehend aus Morphin, Tapentadol, Hydromorphon, Etorphin, Desomorphin, Oxymorphon, Buprenorphin, opioiden Peptiden, aufweisend einen Phenylalaninrest, wie Adrenorphin, Amidorphin, Casomorphin, DADLE ([D-Ala², D-Leu⁵]-Enkephalin), DAMGO ([DD-Ala², N-MePhe⁴, Gly-ol]-Enkephalin), Dermorphin, Endomorphin, Morphiceptin und TRIMU 5 (L-Tyrosyl-N-{[(3-methylbutyl)amino]acetyl}-D-alaninamid); Oripavin, 6-MDDM (6-Methylendihydrodesoxymorphin), Chlornaltrexamin, Dihydromorphin, Hydromorphinol, Methyldesorphin, N-Phenethylnormorphin, RAM-378 (7,8-Dihydro-14-hydroxy-N-phenethylnormorphin), Heterocodein, 7-Spiroindanyloxymorphon, Morphinon, Pentamorphon, Semorphon, Chloromorphid, Nalbuphin, Oxymorphazon, 1-Iodomorphin, Morphin-6-glucuronid, 6-Monoacetylmorphin, Normorphin, Morphin-N-oxid, Cyclorphan, Dextrallorphan, Levorphanol, Levophenacylmorphan, Norlevorphanol, Oxilorphan, Phenomorphan, Furethylnorlevorphanol, Xorphanol, Butorphanol, 6,14-Endoethenotetrahydrooripavin, BU-48 (N-Cyclopropylmethyl-[7α-8α,2',3']-cyclohexano-1'[S]-hydroxy-6,14-endo-ethenotetrahydronororipavin), Cyprenorphin, Dihydroetorphin, Norbuprenorphin, 5'-Guanidinonaltrindol, Diprenorphin, Levallorphan, Meptazinol, Methylnaltrexon, Nalfurafin, Nalmefen, Naloxazon, Naloxon, Nalorphin, Naltrexon, Naltriben, Naltrindol, 6β-Naltrexol-d4, Pseudomorphin, Naloxonazin, Norbinaltorphimin, Alazocin, Bremazocin, Dezocin, Ketazocin, Metazocin, Pentazocin, Phenazocin, Cyclazocin, Hydroxypethidin (Bemidon), Ketobemidon, Methylketobemidon, Propylketobemidon, Alvimopan, Picenadol und pharmazeutisch akzeptable Salze, Hydrate, Solvate, Ester, Prodrugs und Mischungen davon, mehr bevorzugt Morphin, Tapentadol, Buprenorphin, Oxymorphon, Pentazocin, Levorphanol, Hydromorphon, insbesondere Morphin.

5. Pharmazeutische Zusammensetzung nach einem der Ansprüche 1 bis 4, wobei die pharmazeutische Zusammensetzung aus einer Tablette, einer Minitablette, einer Mantel-Kern-Tablette (überzogenen Tablette), einer Zweischichttablette, einer Mehrschichttablette, einer Kapsel, einem Pellet, einem MUPS (Multiple Unit Pellet System), einem Granulat, einem Pulver, insbesondere überzogenen, Zucker-beschichteten und/oder funktionell beschichteten (z. B. enterisch beschichteten) Formen davon ausgewählt ist.

6. Pharmazeutische Zusammensetzung nach einem der Ansprüche 1 bis 5, wobei die Zusammensetzung eine beschichtete Laccase aufweist.

7. Pharmazeutische Zusammensetzung nach einem der Ansprüche 1 bis 6, wobei das Arzneimittel in einer Menge von 0,1 bis 5 000 mg, vorzugsweise 0,5 bis 1 000 mg, insbesondere 1 bis 500 mg, pro Dosierungseinheit enthalten ist.

8. Pharmazeutische Zusammensetzung nach einem der Ansprüche 1 bis 7, wobei die Laccase in einer Menge von 1 bis 1 000 Einheiten, vorzugsweise 10 bis 100 Einheiten, enthalten ist.

9. Pharmazeutische Zusammensetzung nach einem der Ansprüche 1 bis 8, wobei die Zusammensetzung ein weiteres missbrauchsabschreckendes Merkmal aufweist, vorzugsweise aus der Gruppe ausgewählt, bestehend aus einer physikalischen oder chemischen Barriere, insbesondere einer erhöhten Tablettenhärte, einem Antagonisten des Wirkstoffs, einer Aversionskomponente, einem missbrauchsabschreckendem Abgabesystem und einem Prodrug, insbesondere einer physikalischen Barriere oder einer Aversionskomponente, insbesondere einem Geliermittel und/oder einem nicht-gelierenden viskositätssteigernden Mittel.

10. Pharmazeutische Zusammensetzung nach einem der Ansprüche 1 bis 9, wobei die Zusammensetzung eine Matrix aufweist, enthaltend 1 bis 80 Gew.-% eines oder mehrerer hydrophober oder hydrophiler Polymere, vorzugsweise eine Matrix, aufweisend Agar, Alaminsäure, Alginsäure, Carmellose, Carboxymethylcellulose-Natrium, Carbomer, Carrageenan, Chitosan, insbesondere Carboxymethylchitosan, Catechin, Copovidon, Dextrin, Gelatine, Hydroxyethylcellulose, Hydroxypropylcellulose, Hydroxypropylmethylcellulose, Methacrylsäure-Copolymere, Methylcellulose-Derivate, mikrokristalline Cellulose, Polyacrylsäure, Polyalkylenoxid, insbesondere Polyethylenglykol, Polyvinylalkohol, Polyvinylacetat, Povidon, Propylenglycolalginat, ein Polyvinylcaprolactam-Polyvinylacetat-Polyethylenglycol-PfropfCopolymer, Pullulan, Siliciumdioxid, Natriumalginat, Stärke, Vinylpyrrolidon-Vinylacetat-Copolymere oder einen nicht-polymeren Matrixbildner, vorzugsweise mikrokristallines Wachs, Fettalkohole und Fettsäuren, insbesondere Stearylalkohol, Cetylstearylalkohol, Stearinsäure, Palmitinsäure oder Salze und Mischungen davon, Mono-, Di- und Triglyceride gesättigter Fettsäuren mit einer Kettenlänge zwischen 16 und 22 Kohlenstoffatomen und eine Mischung aus solchen Mono-, Di- und Triglyceriden.

11. Pharmazeutische Zusammensetzung nach einem der Ansprüche 1 bis 10, wobei die Zusammensetzung lagerstabil ist, indem sie vorzugsweise weniger als 5 %, insbesondere weniger als 1 %, Laccase-prozessierten Wirkstoff nach 6-monatiger Lagerung bei 25 °C unter trockenen Bedingungen aufweist.

12. Pharmazeutische Zusammensetzung nach einem der Ansprüche 1 bis 11, ferner aufweisend eine Hydrogel-bildende Komponente und/oder einen Vernetzer, vorzugsweise Chitosan und/oder Catechol oder Carboxymethylchitosan und/oder Vanillin.

13. Verfahren zur Herstellung einer pharmazeutischen Zusammensetzung nach einem der Ansprüche 1 bis 12, aufweisend die Schritte des Mischens des Wirkstoffs mit der Laccase und Fertigbearbeitung des Gemischs zu einer pharmazeutischen Zusammensetzung.

14. Verfahren zur Herstellung einer pharmazeutischen Zusammensetzung nach einem der Ansprüche 1 bis 12, aufweisend die Schritte des Bereitstellens des Wirkstoffs und der Laccase in separierter Form und Fertigbearbeiten des separierten Wirkstoffs und der Laccase zu einer pharmazeutischen Zusammensetzung.

15. Pharmazeutische Zusammensetzung nach einem der Ansprüche 1 bis 12 zur Anwendung bei der Behandlung von Schmerzen und/oder Drogensucht.

## Revendications

1. Composition pharmaceutique comprenant un médicament avec un groupe fonctionnel réactif avec la laccase et une laccase (EC 1.10.3.2).

2. Composition pharmaceutique selon la revendication 1, dans laquelle le médicament avec un groupe fonctionnel réactif avec une laccase est choisi dans le groupe comprenant des substances avec un groupe hydroxyle phénolique, des aminophénols, des benzènethiols, des di- ou polyphénols, des phénols substitués par méthoxy, des aminophénols, des diamines, des aminés aromatiques, des polyamines, l'ascorbate, des hydroxyindoles, des aryldiamines, et des anilines, de préférence des substances avec un groupe hydroxyle phénolique.

3. Composition pharmaceutique selon la revendication 1 ou 2, dans laquelle la laccase est choisie dans le groupe de la laccase de *Trametes villosa,* la laccase de *Myceliophthora thermophila*, et la laccase de *Pleurotus ostreatus.*

4. Composition pharmaceutique selon l'une quelconque des revendications 1 à 3, dans laquelle le médicament est un médicament opioïde avec un groupe fonctionnel réactif avec une laccase, de préférence choisi dans le groupe des morphine, tapentadol, hydromorphone, étorphine, désomorphine, oxymorphone, buprénorphine, peptides opioïdes comprenant un résidu phénylalanine, tel que les adrénorphine, amidorphine, casomorphine, DADLE ([D-Ala², D-Leu⁵]-enképhaline), DAMGO (D-Ala², N-MePhe⁴, Gly-ol]-enképhaline), dermorphine, endomorphine, morphiceptine, et TRIMU 5 (L-tyrosyl-N-{[(3-méthylbutyl)amino]acétyl}-D-alaninamide) ; oripavine, 6-MDDM (6-méthylènedihydrodésoxymorphine), chlornaltrexamine, dihydromorphine, hydromorphinol, méthyldésorphine, N-phenéthylnormorphine, RAM-378 (7,8-dihydro-14-hydroxy-N-phénéthylnormorphine), hétérocodéine, 7-spiroindanyloxymorphone, morphinone, pentamorphone, sémorphone, chloromorphide, nalbuphine, oxymorphazone, 1-iodomorphine, morphine-6-glucuronide, 6-monoacétylmorphine, normorphine, morphine-N-oxyde, cyclorphan, dextrallorphan, lévorphanol, lévophénacylmorphan, norlévorphanol, oxilorphan, phénomorphan, furéthylnorlévorphanol, xorphanol, butorphanol, 6,14-endoéthénotétrahydrooripavine, BU-48 (N-cyclopropylméthyl-[7a,8a,2',3']-cyclohexano-1'[S]-hydroxy-6,14-endo-éthénotétrahydronororipavine), cyprénorphine, dihydroétorphine, norbuprénorphine, 5'-guanidinonaltrindole, diprénorphine, lévallorphan, meptazinol, méthylnaltrexone, nalfurafine, nalméfène, naloxazone, naloxone, nalorphine, naltrexone, naltribène, naltrindole, 6β-naltrexol-d4, pseudomorphine, naloxonazine, norbinaltorphimine, alazocine, brémazocine, dézocine, kétazocine, métazocine, pentazocine, phénazocine, cyclazocine, hydroxypéthidine, kétobémidone, méthylkétobémidone, propylkétobémidone, alvimopan, picénadol et des sels pharmaceutiquement acceptables, hydrates, solvates, esters, promédicaments et des mélanges de ceux-ci, plus préférablement la morphine, le tapentadol, la buprénorphine, l'oxymorphone, la pentazocine, le lévorphanol, l'hydromorphone, en particulier la morphine.

5. Composition pharmaceutique selon l'une quelconque des revendications 1 à 4, la composition pharmaceutique étant choisie parmi un comprimé, un minicomprimé, un comprimé du type enrobage-noyau (comprimé enrobé), un comprimé bicouche, un comprimé multicouche, une capsule, un granulé, un système de granulé multiparticulaire (MUPS), un granulat, une poudre, en particulier des formes dragéifiées et/ou fonctionnellement enrobées (par exemple, avec un enrobage entérique) de ceux-ci.

6. Composition pharmaceutique selon l'une quelconque des revendications 1 à 5, la composition comprenant une laccase revêtue.

7. Composition pharmaceutique selon l'une quelconque des revendications 1 à 6, dans laquelle le médicament est contenu en une quantité de 0,1 à 5 000 mg, de préférence 0,5 à 1 000 mg, en particulier 1 à 500 mg, par unité de dose.

8. Composition pharmaceutique selon l'une quelconque des revendications 1 à 7, dans laquelle la laccase est contenue en une quantité de 1 à 1 000 unités, de préférence 10 à 100 unités.

9. Composition pharmaceutique selon l'une quelconque des revendications 1 à 8, la composition comprenant un élément anti-abus supplémentaire, de préférence choisi dans le groupe d'une barrière physique ou chimique, en particulier une dureté de comprimé augmentée, un antagoniste de médicament, un composant d'aversion, un système d'administration anti-abus et un promédicament, en particulier une barrière physique ou un composant d'aversion, en particulier un agent gélifiant et/ou un agent augmentant la viscosité non gélifiant.

10. Composition pharmaceutique selon l'une quelconque des revendications 1 à 9, la composition comprenant une matrice contenant 1 à 80 % en poids d'un ou plusieurs polymères hydrophobes ou hydrophiles, de préférence une matrice comprenant la gélose, l'acide alamique, l'acide alginique, la carmellose, la carboxyméthylcellulose sodique, un carbomère, la carraghénine, le chitosane, en particulier le carboxyméthylchitosane, le catéchol, la copovidone, la dextrine, la gélatine, l'hydroxyéthylcellulose, l'hydroxypropylcellulose, l'hydroxypropylméthylcellulose, des copolymères d'acide méthacrylique, des dérivés de méthylcellulose, la cellulose microcristalline, l'acide polyacrylique, le poly(oxyde d'alkylène), en particulier le polyéthylène glycol, l'alcool polyvinylique, le poly(acétate de vinyle), la povidone, l'alginate de propylène glycol, un copolymère greffé polyvinylcaprolactame-poly(acétate de vinyle)-polyéthylène glycol, le pullulane, le dioxyde de silicium, l'alginate de sodium, l'amidon, des copolymères de vinylpyrrolidone-acétate de vinyle ou d'un agent de formation de matrice non polymère, de préférence la cire microcristalline, des alcools gras et des acides gras, en particulier l'alcool stéarylique, l'alcool cétylstéarylique, l'acide stéarique, l'acide palmitique ou des sels et des mélanges de ceux-ci, des mono-, di- et triglycérides d'acides gras saturés ayant une longueur de chaîne comprise entre 16 et 22 atomes de carbone et un mélange de tels mono-, di- et triglycérides.

11. Composition pharmaceutique selon l'une quelconque des revendications 1 à 10, la composition étant stable à la conservation, de préférence comprenant moins de 5 %, en particulier moins de 1 %, de médicament traité par laccase après 6 mois de conservation à 25 °C dans des conditions sèches.

12. Composition pharmaceutique selon l'une quelconque des revendications 1 à 11, comprenant en outre un composant formant un hydrogel et/ou un agent de réticulation, de préférence le chitosane et/ou le catéchol ou le carboxyméthylchitosane et/ou la vanilline.

13. Procédé de fabrication d'une composition pharmaceutique selon l'une quelconque des revendications 1 à 12 comprenant les étapes de mélange du médicament avec la laccase et finition du mélange sous la forme d'une composition pharmaceutique.

14. Procédé de fabrication d'une composition pharmaceutique selon l'une quelconque des revendications 1 à 12 comprenant les étapes de fourniture du médicament et de la laccase dans une forme séparée et de finition du médicament et de la laccase séparés sous la forme d'une composition pharmaceutique.

15. Composition pharmaceutique selon l'une quelconque des revendications 1 à 12 pour utilisation dans le traitement de la douleur et/ou de la toxicomanie.
